# EUROPEAN PATENT APPLICATION

(11) **EP 1 229 000 A2**
(43) Date of publication of application: **07.08.2002**
(21) Application number: 02075811.6
(22) Date of filing: 10.11.1998
(51) Int. Cl.: C01B 21/083, C07C 17/386, B01D 3/36

(54) **Process for purifying perfluorinated products**

(30) Priority: 10.11.1997 US 64993 P; 20.05.1998 US 86146 P; 09.11.1998 US 189322
(62) Divisional of application: 98958520.3
(71) Applicant: E.I. DU PONT DE NEMOURS AND COMPANY, Wilmington Delaware 19898 (US)
(72) Inventor: The designation of the inventor has not yet been filed
(74) Representative: Matthews, Derek Peter

(57) **Abstract**

A process for separating nitrogen trifluoride (NF₃) from a first mixture of nitrogen trifluoride (NF₃) and tetrafluoromethane (PFC-14) wherein the amount of nitrogen trifluoride (NF₃) in the first mixture is in excess of the amount of nitrogen trifluoride (NF₃) in an azeotropic or azeotrope-like composition of nitrogen trifluoride (NF₃) and tetrafluoromethane (PFC-14), comprising:
a.) distilling the first mixture to form an azeotropic or azeotrope-like composition of nitrogen trifluoride (NF₃) and tetrafluoromethane (PFC-14) as a second mixture, and
b.) recovering the second mixture as a distillation column overhead stream, and nitrogen trifluoride (NF₃) as a distillation column bottom stream.

## Description

### BACKGROUND OF THE INVENTION

Various gaseous fluorine-containing compounds are utilized in manufacturing processes that plasma-etch silicon-type materials in order to fabricate semiconductor devices. A major use of tetrafluoromethane (CF₄ or PFC-14) is for plasma etching during semiconductor device fabrication. Plasma etchants interact with the surface of the integrated circuit wafer, modifying it so as to lay down the electrical pathways and providing for the surface functionalities that define the integrated surface. A major use of nitrogen trifluoride (NF₃) is as a "chemical vapor deposition" (CVD) chamber cleaning gas in semiconductor device manufacture. CVD chamber cleaning gases are used to form plasmas which interact with the internal surfaces of semiconductor fabrication equipment to remove the various deposits that accumulate over time.

Perfluorinated chemicals such as PFC-14 and NF₃ that are used in semiconductor manufacturing applications as etchant or cleaning gases are more commonly referred to as "electronic gases". Electronic gases having high purity are critical for such semiconductor device manufacture applications. It has been found that even very small amounts of impurities in these gases that enter semiconductor device manufacturing tools can result in wide line width and thus less information per device. Moreover, the presence of these impurities, including but not limited to particulates, metals, moisture, and other halocarbons in the plasma etchant or cleaning gases, even when only present in the part-per-million level, increases the defect rate in the production of these high-density integrated circuits. As a result, there has been increasing demand for higher purity etchant and cleaning gases, and an increasing market value for the materials having the required purity. Identification of offending components and methods for their removal consequently represents a significant aspect of preparing the fluorine-containing compounds for these applications.

These etchant and cleaning gases are not fully consumed by semiconductor manufacturing processes, but typically exit the integrated circuit fabrication equipment in finite concentrations. These fabrication equipment exhaust streams not only contain varying amounts of the unreacted perfluorinated etchant and cleaning gases, but may also contain a variety of reaction products and air components, which include but are not limited to hydrogen fluoride (HF), tetrafluoroethylene (C₂F₄ or PFC-1114), methyl fluoride (CH₃F or HFC-41), trifluoromethane (CHF₃ or HFC-23), chlorotrifluoromethane (CClF₃ or CFC-13), nitrogen, oxygen, carbon dioxide, water, methane, ethane, propane and nitrous oxide (N₂O). A variety of other fluorinated compounds are also used in semiconductor manufacturing applications, including hexafluoroethane (C₂F₆ or PFC-116), octafluorocyclobutane (cyclic C₄F₈ or PFC-C318), octafluoropropane (C₃F₈ or PFC-218), sulfur hexafluoride (SF₆), pentafluoroethane (C₂HF₅ or HFC-125), trifluoromethane (CHF₃ or HFC-23), tetrafluoroethane (C₂H₂F₄, or HFC-134a or HFC-134) and difluoromethane (CH₂F₂ or HFC-32), and the exhaust streams coming off these processes are frequently combined with the exhaust streams from the PFC-14 and NF₃ processes. The resulting combined exhaust stream consequently may contain a wide range of compounds and concentrations.

Considerable effort is underway to develop ways and means to capture the fluorinated compounds present in such equipment exhaust streams and to develop options for their disposition. A preferred disposition option is to repurify certain of the fluorinated components from these streams for reuse. Separation of several of these valuable fluorinated compounds is made difficult due to the variety of fluorinated compounds that might be present in the combined exhaust gas stream from any given manufacturing site, and due to non-ideal interactions that exist between several of these compounds. For example, several of these compounds form azeotropic or azeotrope-like compositions with other compounds in these streams, making separation by conventional distillation at least difficult, if not impossible.

The present invention provides compositions and distillation processes for removing fluorinated impurities from an impure stream comprising at least one of PFC-14 and NF₃ so as to produce a purified PFC-14 and/or NF₃ product. The present processes are simple to carry out and are effective for obtaining either of these two compounds in high purity and with high degrees of recovery.

### SUMMARY OF THE INVENTION

The present invention comprises NF₃ substantially free of impurities, containing less than 10 parts-per-million molar of impurities. The present invention further comprises NF₃ containing less than 10 parts-per-million molar PFC-14.

The present invention further comprises azeotropic compositions consisting essentially of: NF₃ and PFC-14; hydrogen chloride and PFC-14; NF₃ and hydrogen chloride; nitrous oxide and trifluoromethane; and nitrous oxide and hydrogen chloride.

The present invention further comprises a process for separating at least one of PFC-14 and NF₃ from a first mixture comprising PFC-14, NF₃, and optionally other fluorinated compounds, comprising the steps of:
contacting at least one entraining agent with the first mixture to form a second mixture,
distilling the second mixture, and
recovering at least one of PFC-14 and NF₃ that is substantially free of at least one of the other fluorinated components of the first mixture.

### BRIEF DESCRIPTION OF THE DRAWINGS

Fig 1 is a schematic diagram of a distillation system that can be used for practicing an aspect of the present process.
Fig 2 is a schematic diagram of a distillation system that can be used for practicing an aspect of the present process
Fig 3 is a graphical representation of an azeotropic and azeotrope-like composition consisting essentially of PFC-14 and NF₃ at a temperature of about -70°C.
Fig 4 is a graphical representation of an azeotropic and azeotrope-like composition consisting essentially of PFC-14 and HCl at a temperature of about -76°C.
Fig 5 is a graphical representation of an azeotropic and azeotrope-like composition consisting essentially of NF₃ and HCl at a temperature of about -78°C.
Fig 6 is a graphical representation of an azeotropic and azeotrope-like composition consisting essentially of N₂O and HFC-23 at a temperature of about -70°C.
Fig 7 is a graphical representation of an azeotropic and azeotrope-like composition consisting essentially of HCl and N₂O at a temperature of about -30°C.

### DETAILED DESCRIPTION

PFC-14 and NF₃, in their separate and pure states, exhibit properties that are valued for integrated circuit manufacturing and are typically used in a variety of the related manufacturing steps. The desire for greater precision and consistency of the effect such compounds have during integrated circuit manufacture has made extremely high purities critical for such applications. The presence of any other compounds in the PFC-14 or NF₃ is objectionable for most of the intended uses. It should be recognized that any one of PFC-14 and NF₃ might in themselves be considered an impurity if present in the product stream of the other. For example, even a 1 parts-per-million-molar concentration of PFC-14 would be considered an impurity in NF₃ where that NF₃ is to be sold as a cleaning agent product. Similarly, even a 1 parts-per-million-molar concentration of NF₃ would be considered an impurity in PFC-14 where that PFC-14 is to be sold as an etchant product. Processes that allow for manufacture of PFC-14 or NF₃ products having purities that approach 99.999 molar percent purity are desirable, but processes that provide at least 99.9999 molar percent purity for electronic gas applications are preferred. Analytical methods for gauging such low concentrations of impurities in PFC-14 and NF₃ products are available. For example, methods for analyzing low concentrations of PFC-14 and other impurities in an NF₃ product is disclosed in the 1995 SEMI standards, pages 149-153, SEMI C3.39.91-Standard for Nitrogen Trifluoride. Alternately, techniques for analyzing the concentration of PFC-14 and other impurities at low concentrations in PFC-116, but which may also be applied to analysis of NF₃ and PFC-14 products, are disclosed in "Examining Purification and Certification Strategies for High-Purity C₂F₆ Process Gas", Micro Magazine, April 1998, page 35.

Conventional processes for manufacturing NF₃ often produce PFC-14 as a component in the NF₃ product stream. Because conventional processes are not able to separate the PFC-14 from the NF₃ product, NF₃ products containing less than about 10 ppm-molar PFC-14 are not available in spite of the desirability of lower concentrations of PFC-14 in said NF₃ product.

Furthermore, during their use in integrated circuit manufacturing processes, neither the PFC-14 nor NF₃ electronic gases are completely consumed, and at least some amounts of these compounds remain in the manufacturing process equipment exhaust stream. This exhaust stream may contain a variety of additional impurities such as byproduct hydrogen chloride (HCl), hydrogen fluoride (HF), and a variety of fluorinated compounds, among others. In a typical manufacturing facility the exhaust streams from the various PFC-14 and NF₃ equipment are mixed not only with each other, but with the exhaust streams from equipment employing a variety of other fluorocarbon chemicals. Typically, this results in a stream containing a wide range of PFC-14, NF₃, and other fluorinated impurities in a wide range of concentrations. Typically this exhaust stream also contains relatively high volume concentrations, typically greater than 50 volume %, of inert carrier gases such as air, helium or nitrogen.

Concerns over possible environmental impact of such materials and the high value-in-use of these materials has prompted a search for methods of recovering PFC-14 or NF₃ from said exhaust streams of such processes. Conventional methods of recovering the components from such streams typically involve water washing the exhaust stream to remove the HF and HCl, then drying the stream using a variety of methods. Conventional methods for separating and recovering the fluorinated compounds from the large concentrations of inert carrier gases include use of semi-permeable membranes or adsorption of the fluorinated compounds into liquid solvents. However, a wide range of fluorinated organic and inorganic compounds typically still remain in the captured stream after such processing steps, making any PFC-14 or NF₃ contained within unsuitable for reuse as electronic gases.

The ability to separate and recover a NF₃ product that is substantially free of PFC-14 and other fluorinated impurities, particularly where the PFC-14 concentration in said NF₃ product is preferably less than 3, more preferably less than 1 ppm-molar, is of considerable commercial interest. The ability to separate and recover a PFC-14 product that is substantially free of fluorinated impurities is also of considerable commercial interest.

Many of the fluorinated compounds used or that are produced in semiconductor process operations are extremely close-boiling in their separated and pure states or otherwise exhibit non-ideal behavior such that their relative volatility approaches or even becomes 1.0 compared to any one of PFC-14 or NF₃. Compounds whose relative volatilities approach or equal 1.0 compared to PFC-14 or NF₃ make their separation from said PFC-14 or NF₃ by conventional distillation difficult. Separation of such mixtures is particularly problematic where it is desired that the recovered PFC-14 or NF₃ product be substantially free of other fluorinated compounds and where the PFC-14 or NF₃ product needs to be recovered from a first mixture with high recovery efficiency.

The present inventors have found that PFC-14 and NF₃ may be separated from a variety of fluorinated compounds and each other such that said PFC-14 or NF₃ may be recovered substantially free of other fluorinated compounds with high recovery efficiency of said PFC-14 or NF₃, and such that the separation is effected in an economical manner, by distilling a mixture comprising at least one of PFC-14 and NF₃ in the presence of an entraining agent that interacts in a non-ideal manner with the mixture. Entraining agents act in a manner so as to increase or decrease the volatility of the PFC-14 or NF₃ relative to a fluorinated impurity. These thus allow a PFC-14 or NF₃ product that is substantially free of fluorinated impurities to be separated and recovered from the initial mixture comprising PFC-14 or NF₃ by distillation.

In one aspect of this invention, an effective amount of an entraining agent is fed to a distillation column at a point equal to, or higher than, that at which the PFC-14 or NF₃ containing mixture is being fed. The entraining agent acts in a non-ideal manner with at least one of the PFC-14, NF₃, or their respective fluorinated impurities such that the relative volatility between the desired PFC-14 or NF₃ product and their respective fluorinated impurities is increased. By distilling the mixture, the fluorinated impurity may be separated from the desired PFC-14 or NF₃ product.

By effective amount of entraining agent is meant an amount of at least one entraining agent which, in the presence of a desired product and fluorinated impurity, causes the volatility of the fluorinated impurity to increase or decrease relative to the desired product sufficiently to allow separation by distillation of the impurity from the desired product. Further, by effective amount of entraining agent is meant an amount which, in the presence of a desired product and fluorinated impurity, results in the formation of a lower- or higher-boiling azeotropic or azeotrope-like composition or otherwise causes the volatility of the fluorinated impurity to increase or decrease relative to the desired product sufficiently to allow separation by distillation of the impurity from the desired product. This definition includes where the effective amount may vary depending on the pressure applied to the composition so long as the azeotrope or azeotrope-like compositions or changes in relative volatility continue to exist.

In one embodiment of the present invention, an effective amount of entraining agent is added to the PFC-14 or NF₃ containing mixture and the resulting mixture distilled under conditions such that azeotropic or azeotrope-like compositions are formed. The entraining agent acts in a non-ideal manner with at least one of the PFC-14, NF₃, or their respective fluorinated impurities such that the relative volatility between the desired PFC-14 or NF₃ product and their respective impurities is increased. By distilling the mixture, the fluorinated impurity may be separated from the desired PFC-14 or NF₃ product.

It is also recognized in the art that when the relative volatility of a system, for example, a mixture comprising any one of PFC-14 and NF₃ and at least one other compound approaches 1.0, that is for example when the relative volatility is 0.98 or 1.02, such defines the system as forming an azeotrope-like composition. When the relative volatility is equal to 1.0, such defines the system as forming an azeotrope.

To determine the relative volatility of any given two compounds, a method known as the PTx Method may be used. In this procedure, the total absolute pressure in a cell of known volume is measured at a constant temperature for various compositions of the two compounds. Use of the PTx Method is described in greater detail in "Phase Equilibrium in Process Design", Wiley-Interscience Publisher, 1970, written by Harold R. Null, on pages 124 to 126.

These measurements can be converted into equilibrium vapor and liquid compositions in the PTx cell by using an activity coefficient equation model, such as the Non-Random, Two-Liquid (NRTL) equation, to represent liquid phase nonidealities. Use of an activity coefficient equation, such as the NRTL equation is described in greater detail in "The Properties of Gases and Liquids," 4th edition, published McGraw Hill, written by Reid, Prausnitz and Poling, on pages 241 to 387, and in "Phase Equilibria in Chemical Engineering," published by Butterworth Publishers, 1985, written by Stanley M. Walas, pages 165 to 244.

Without wishing to be bound by any theory or explanation, it is believed that the NRTL equation, together with the PTx cell data, can sufficiently predict the relative volatilities of PFC-14, NF₃, and other compounds, and combinations and mixtures thereof, and can therefor predict the behaviour of PFC-14, NF₃ and other and mixtures in multi-stage separation equipment such as distillation columns.

By conventional distillation is meant that only the relative volatilities of the components of the mixture to be separated are used to separate the components.

By substantially free or substantially pure is meant that any given fluorinated compound other than the PFC-14 or NF₃ in the respective PFC-14 or NF₃ product is less than 10 parts-per-million-by-volume (ppm-volume) or 10 parts-per-million-molar (ppm-molar), more preferably less than 1 ppm-volume or 1 ppm-molar, most preferably less than 100 parts-per-billion-by-volume (ppbv) or 100 parts-per-billion-molar (ppb-molar). Alternately, by substantially free or substantially pure is meant that any given fluorinated compound other than the PFC-14 or NF₃ in the respective PFC-14 or NF₃ product is less than 10 parts-per-million-by-weight (ppm-weight), more preferably less than 1 ppm-weight, most preferably less than 100 parts-per-billion-by-weight (ppb-weight).

By high recovery efficiency is meant that greater than 90%, most typically greater than 95% of the PFC-14 or NF₃ in a first mixture is recovered as product substantially free of a specific fluorinated impurity.

By impurity is meant any fluorinated compound other than the PFC-14 or NF₃ that may be present in the respective PFC-14 or NF₃ product.

By azeotropic or azeotrope composition is meant a constant-boiling mixture of two or more substances that behaves as a single substance. One way to characterize an azeotropic composition is that the vapor produced by partial evaporation or distillation of the liquid has the same composition as the liquid from which it is evaporated or distilled, i.e., the mixture distills/refluxes without compositional change. Constant-boiling compositions are characterized as azeotropic because they exhibit either a maximum or minimum boiling point, as compared with that of the non-azeotropic mixture of the same components. Azeotropic compositions are also characterized by a minimum or a maximum in the vapor pressure measurements relative to the vapor pressure of the neat components in a PTx cell as a function of composition at a constant temperature.

By azeotrope-like is meant a composition that has a constant-boiling characteristic or a tendency not to fractionate upon boiling or evaporation. Therefore, the composition of the vapor formed is the same as or substantially the same as the original liquid composition. During boiling or evaporation, the liquid composition, if it changes at all, changes only to a minimal or negligible extent. An azeotrope-like composition can also be characterized by the area that is adjacent to the maximum or minimum vapor pressure in a plot of composition vapor pressure at a given temperature as a function of mole fraction of components in the composition. A composition is azeotrope-like if, after about 50 weight percent of an original composition is evaporated or boiled off to produce a remaining composition, the change between the original composition and the remaining composition is typically no more than about 6 weight% and typically no more than about 3 weight% or less relative to the original composition.

By high-boiling azeotrope is meant that an azeotropic or azeotrope-like composition boils at a higher temperature at any given pressure than any one of the compounds that comprise it would separately boil at that pressure. Alternately, by high-boiling azeotrope is meant any azeotropic or azeotrope-like composition that has a lower vapor pressure at any given temperature than any one of the compounds that comprise it would separately have at that temperature.

By low-boiling-azeotrope is meant that an azeotropic or azeotrope-like composition boils at a lower temperature at any given pressure than any one of the compounds that comprise it would separately boil at that pressure. Alternately, by low-boiling azeotrope is meant any azeotropic or azeotrope-like composition that has a higher vapor pressure at any given temperature than the vapor pressure of any one of the compounds that comprise the azeotrope would separately have at that temperature.

It is possible to characterize an azeotropic or azeotrope-like composition as a substantially constant-boiling admixture that may appear under many guises, depending upon the conditions chosen, by several criteria:
* The composition can be defined as an azeotrope of two compounds because the term "azeotrope" is at once both definitive and limitative, and requires effective amounts of those two or more compounds for this unique composition of matter which can be a constant-boiling composition.
* It is well known by those skilled in the art, that, at different pressures, the composition of a given azeotrope or azeotrope-like composition will vary at least to some degree, as will the boiling point temperature. Thus, an azeotropic or azeotrope-like composition of two compounds represents a unique type of relationship but with a variable composition which depends on temperature and/or pressure. Therefore, compositional ranges, rather than fixed compositions, are often used to define azeotropes and azeotrope-like compositions.
* An azeotrope or azeotrope-like composition of two compounds can be characterized by defining compositions characterized by a boiling point at a given pressure, thus giving identifying characteristics without unduly limiting the scope of the invention by a specific numerical composition, which is limited by and is only accurate as the analytical equipment available.

It is recognized in the art that both the boiling point and the weight (or mole) percentages of each component of the azeotropic composition may change when the azeotrope or azeotrope-like liquid composition is allowed to boil at different pressures. Thus, an azeotropic or an azeotrope-like composition may be defined in terms of the unique relationship that exists among components or in terms of the exact weight (or mole) percentages of each component of the composition characterized by a fixed boiling point at a specific pressure.

By entraining agent is meant any compound that, when added to a first mixture, interacts with the components in that first mixture in a way that changes the relative volatilities of the components in the first mixture to each other such that those components may then be separated by distillation.

By azeotropic distillation is meant a process in which a distillation column is operated under conditions to cause an azeotropic or azeotrope-like composition to form, and the formation thereof changes the relative volatility of the components to each other such that the components may be separated by distillation. Azeotropic distillations may occur where only the components of the mixture to be separated are distilled, or where an entraining agent is added that forms an azeotrope with one or more of the components of the initial mixture. Entraining agents that act in this manner, that is to say, that form an azeotrope with one of more of the components of the mixture to be separated thus facilitating the separation of those components by distillation, are more commonly called azeotroping agents or azeotropic entraining agents.

By extractive distillation is meant a process in which an entraining agent is introduced at an upper feed point of the distillation column, whereas the mixture requiring separation is introduced at the same or preferably a relatively lower feed point of the column than the entraining agent. The entraining agent passes downwardly through trays or packing located in the column and exits the column bottoms with one or more components of the mixture to be separated. While in the presence of the entraining agent, at least one of the components to be separated becomes relatively more volatile compared to the other fluorinated compound(s) of the mixture, with that more volatile fluorinated component of the initial mixture exiting the column overheads. Entraining agents which are fed to a distillation column at a point equal to, or higher than, the mixture to be separated and which pass down through the column thus enabling a separation by distillation, are more commonly called extractive agents or extractants.

In conventional, azeotropic, or extractive distillations, the overhead or distillate stream exiting the column may be condensed using conventional reflux condensers. At least a portion of this condensed stream can be returned to the top of the column as reflux, and the remainder recovered as product or for optional processing. The ratio of the condensed material which is returned to the top of the column as reflux to the material removed as distillate is commonly referred to as the reflux ratio. The compounds and entraining agent exiting the column as distillate or distillation bottoms stream can then be passed to a stripper or second distillation column for separation by using conventional distillation, or may be separated by other methods. If desired, the entraining agent may then be recycled back to the first distillation column for reuse.

The specific conditions which can be used for practicing the invention depend upon a number of parameters, such as the diameter of the distillation column, feed points, number of separation stages in the column, among others. The operating pressure of the distillation system may range from about 0.10 (15) to 3.45 MPa (500 psia), normally about 0.34 (50) to 2.76 MPa (400 psia). Typically, an increase in the extractant or azeotroping agent feed rate relative to the feed rate of the mixture to be separated causes an increase in the purity of the product to be recovered with regard to those compound(s) being removed. Normally, increasing the reflux ratio results in increased distillate stream purity, but generally the reflux ratio ranges between 1/1 to 200/1. The temperature of the condenser, which is located adjacent to the top of the column, is normally sufficient to substantially fully condense the distillate that is exiting from the top of the column, or is that temperature required to achieve the desired reflux ratio by partial condensation.

The problems associated with conventional distillation can be solved by a distillation process using entraining agents. This method may be employed when the components of the mixture have relative volatilities that do not allow effective separation of the components by conventional distillation. In distillation using entraining agents, an entraining agent is added that causes the relative volatilities of the components in the starting mixture to be altered such that the relative volatility becomes sufficient to permit separation of the components. The difficulty in applying this method is that there is no known way, short of experimentation, of predicting which if any compound will be an effective entraining agent.

The first mixture can be obtained from any suitable manufacturing process or source which produces or generates a mixture comprising at least one of PFC-14 and NF₃. For example: PFC-14 may be produced by reacting a chlorocarbon or chlorofluorocarbon with HF; NF₃ may be produced by reacting ammonia (NH₃) with elemental fluorine (F₂). Alternately, the first mixture can be obtained from any suitable manufacturing process that uses any one of PFC-14 or NF₃ and desires to recover said PFC-14 or NF₃ from said process. Methods such as conventional distillation would then be used for removing inert carrier gases and for reducing initial amounts of other fluorinated impurities. The PFC-14 or NF₃ containing stream then may be processed in accordance with the inventive process for recovering and purifying PFC-14 or NF₃.

NF₃ and PFC-14, in their separated and pure states have normal boiling points of -129.1 and -128.1°C. These close boiling points alone would make efficient separation of NF₃ and PFC-14 extremely difficult by conventional distillation. However, mixtures of NF₃ and PFC-14 in addition form azeotropic or azeotrope-like compositions which makes their complete separation by conventional distillation impossible.

It is desirable to purify and recover separate product streams of each of PFC-14 and NF₃ that are substantially free of other fluorinated components. The present inventors have found that NF₃ and PFC-14 form an azeotropic or azeotrope-like compositions over a range of temperatures and pressures, and that NF₃ and PFC-14 can be partially purified by using said NF₃/PFC-14 azeotropic and azeotrope-like compositions. For example, a conventional distillation column can be operated at a pressure and temperature that causes an azeotropic or azeotrope-like composition to form. If the quantity of NF₃ versus PFC-14 in the column is greater than that in the azeotropic or azeotrope-like composition, a NF₃ product can be removedfrom the bottom of the column with the PFC-14 concentration in it reduced compared to the PFC-14 concentration in the NF₃/PFC-14 containing mixture initially fed or charged to the distillation column, while the azeotropic or azeotrope-like composition is removed from the top of the column. Conversely, if the quantity of PFC-14 versus NF₃ in the column is greater than that in the azeotropic or azeotrope-like composition, a PFC-14 product can be removed from the bottom of the column with the NF₃ concentration in it reduced compared to the NF₃ concentration in the NF₃/PFC-14 containing mixture initially fed or charged to the distillation column, while the azeotropic or azeotrope-like composition is removed from the top of the column. Obtaining a NF₃ product stream with the concentration of PFC-14 in it reduced compared to a first mixture with PFC-14 or obtaining a PFC-14 product stream with the concentration of NF₃ reduced compared to a first mixture with NF₃ in a single distillation would require starting with a composition higher in NF₃ or PFC-14 respectively than the azeotrope, but some portion of the NF₃ or PFC-14 respectively would necessarily remain as the NF₃/PFC-14 azeotrope.

The NF₃ can be partially separated from the PFC-14 by a series of multiple distillations performed at alternately higher and lower pressures that comprises forming these low-boiling, high-pressure azeotropes or azeotrope-like NF₃ and PFC-14 containing compositions within conventional distillation columns by taking advantage of changes in the NF₃/PFC-14 azeotrope composition that occur with pressure. By taking the overhead distillate from a column that is operated under conditions such that one component is in excess to the azeotrope (a first distillation), then feeding that distillate to a column operated under conditions such that the other component is in excess of the azeotrope (a second distillation), then feeding the distillate from the second distillation to a column where we repeat the sequence, that is, where the next column is again operated under conditions such that the first component is in excess, it is possible to produce a bottoms product of NF₃ from one distillation and of PFC-14 from a second distillation each of which has had the concentration of the other component reduced compared to a first mixture comprising NF₃ and PFC-14. This separation by "pressure-swing" distillation is possible only due to the unusual composition change of the azeotrope with pressure or temperature.

However, in these cases, where only the relative volatility of the NF₃/PFC-14 azeotrope compared to NF₃ or PFC-14 present in excess of the azeotrope is used as the basis for their separation, such separations would require tall and expensive distillation columns, and it would still be impossible to produce a substantially pure NF₃ or PFC-14 product from a NF₃/PFC-14 containing starting mixture.

The present inventors have found that PFC-14 may be separated from a variety of fluorinated compounds to produce a substantially pure NF₃ by the use of entraining agents in an azeotropic distillation process. For example, PFC-14 can be efficiently separated from NF₃ by the addition of HCl to a first mixture as an entraining agent, forming a second mixture, distilling the second mixture under conditions so as to form a azeotropic or azeotrope-like composition comprising HCl and PFC-14, and distilling the PFC-14 and HCl azeotropic composition overhead from the column. The PFC-14 in the distillate may then optionally be separated from the HCl, for example by water washing or by use of a semi-permeable membrane which preferentially allows HCI to pass through versus the PFC-14 or by other commonly known techniques, and the PFC-14 recovered as substantially pure product.

HCl forms azeotropic or azeotrope-like compositions with a number of the fluorinated compounds typically found in the exhaust gas obtained from integrated manufacturing processes. Examples of azeotropic or azeotrope-like compositions that may be formed between HCl and these fluorinated compounds are shown in Table 1. In Table 1, mixtures comprising compound "A" and compound "B" form low-boiling azeotropic or azeotropic-like compositions comprising the specified moles of compound "A" and the specified moles of compound "B" and having the specified pressure at the indicated temperature

**Table 1**

| Compound "A" | Moles "A" | Compound "B" | Moles "B" | Temp °C | Pressure | |
|---|---|---|---|---|---|---|
| | | | | | MPa | (psia) |
| HCl | 9.6 | PFC-14 | 90.4 | -65 | 2.14 | (311) |
| HCl | 6.8 | NF₃ | 93.2 | -78 | 1.35 | (196) |
| HCl | 63.5 | PFC-116 | 36.5 | -20 | 2.21 | (321) |
| HCl | 89.4 | PFC-218 | 10.6 | -20 | 1.56 | (226) |
| HCl | 3.2 | CO₂ | 96.8 | -15 | 2.29 | (332) |
| HCl | 70.4 | SF₆ | 29.6 | -15 | 2.21 | (321) |
| HCl | 17.3 | N₂O | 82.7 | -20 | 1.81 | (263) |
| HCl | 52.6 | Ethane | 47.4 | -25 | 1.68 | (244) |
| HCl | 68.3 | CF₂=CF₂ | 31.7 | -20 | 1.80 | (261) |

The present inventors have found that HCl and PFC-14 form azeotropic or azeotrope-like compositions over a range of temperatures and pressure. Surprisingly, the HCl/PFC-14 azeotropic or azeotrope-like compositions have the lowest boiling point temperature at any given pressure, and the highest vapor pressure at any given temperature of either the pure components or the azeotropic compositions comprising HCl and the pure components shown in Table 1. By distilling mixtures comprising PFC-14 in the presence of HCl, and distilling under conditions so as to form the PFC-14/HCl azeotrope, the HCl/PFC-14 azeotropic or azeotrope-like composition may be recovered as overhead distillate that is substantially free of the other compounds while the other compounds are recovered from the column bottoms. HCl may optionally then be separated from the PFC-14, for example, by water washing or by use of a semi-permeable membrane or other commonly known techniques and the PFC-14 recovered.

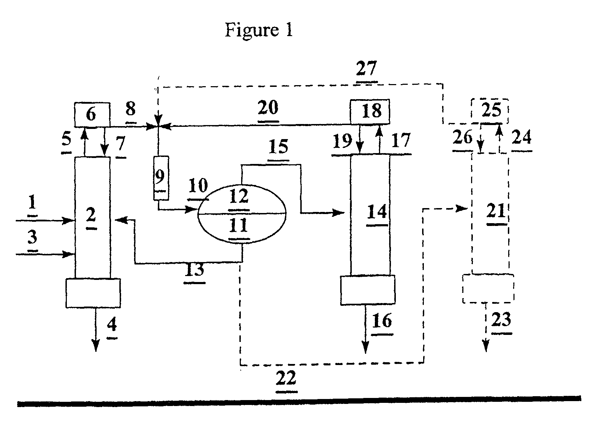

Certain aspects of the present invention can be better understood by reference to Figure 1. Figure 1 schematically illustrates a system that can be used for performing one aspect of the inventive distillation process. A first mixture comprising NF₃ and PFC-14 is supplied via conduit 1 to distillation column 2. At least one azeotropic agent, e.g., HCl, is supplied via conduit 3 to distillation column 2. The entraining agent may alternately be mixed with the NF₃ and PFC-14-containing mixture prior to the distillation column and simultaneously fed in via conduit 1. The column is operated under conditions such that a lower-boiling azeotropic or azeotrope-like mixture is formed between the PFC-14 and azeotropic agent. NF₃ substantially free of PFC-14 is recovered out the distillation column bottoms via conduit 4. The distillate from the column comprising PFC-14 and the azeotropic agent exits via conduit 5 and is fed to the column condenser 6. Part of the condensed distillate is returned to the distillation column as reflux via conduit 7. The remainder of the condensed distillate comprising PFC-14 and the azeotropic agent may be recovered, or optionally separated to separately recover the PFC-14 and azeotropic agent.

For example, where HCl is used as the azeotropic agent for this process, the HCl may be removed from the PFC-14 distillate product by water washing then drying the PFC-14 stream, thus recovering PFC-14 substantially free of either NF₃ or HCI. Alternately, where HCI is used as the azeotroping agent for this process, a stream comprising an azeotropic or azeotrope-like composition comprising HCI and PFC-14 may be sent via conduit 8 to cooler 9 then to a suitable separation unit 10, e.g., a membrane separation unit, where the HCl-containing mixture is separated into two streams, one higher in concentration in HCl (HCl-enriched) 11 and the other lower in HCl concentration (HCl-depleted) 12 compared to the column 2 distillate mixture. The HCl-enriched stream 11 may be recycled back via conduit 13 to be mixed with the feed to distillation column 2. The HCl-depleted stream 12 may be sent to distillation column 14 via conduit 15, where column 14 is operated under conditions to form an azeotrope comprising HCl and PFC-14. Because the HCl and PFC-14 containing stream fed to this second distillation is higher in PFC-14 concentration versus the HCl and PFC-14 azeotrope, by operating the column under conditions such that an azeotropic or azeotrope-like mixture of HCl and PFC-14 is formed, PFC-14 substantially free of HCl will exit the column bottoms via conduit 16. The column distillate comprising HCl and PFC-14 exit overhead column 14 and is fed via conduit 17 to condenser 18. At least part of the liquid condensate is returned via conduit 19 to the column as reflux, while the remainder is recycled via conduit 20 to mix with the streams being fed to separator 10.

In Figure 1, the HCl-enriched stream 11 may be alternately sent to distillation column 21 via conduit 22, where column 21 is operated under conditions to form an azeotrope comprising HCl and PFC-14. Because the HCl and PFC-14-containing stream fed to this second distillation is higher in HCl concentration versus the HCl and PFC-14 azeotropic or azeotrope-like composition, by operating the column under conditions such that an azeotropic or azeotrope-like mixture of HCl and PFC-14 is formed, HCl substantially free of PFC-14 will exit the column bottoms via conduit 23. The column distillate comprising HCl and PFC-14 exit overhead column 21 and is fed via conduit 24 to condenser 25. At least part of the liquid condensate is returned via conduit 26 to the column as reflux, while the remainder is recycled via conduit 27 to mix with the feed steams to separator 10.

In another aspect of the current invention, we have found that NF₃ and PFC-14 may be separated from each other and other fluorinated impurities by the use of entraining agents in an extractive distillation process. Suitable entraining agents that may be used as extractants for the separation of NF₃ and PFC-14 include: hydrocarbons, hydrofluorocarbons, hydrochlorofluorocarbons, hydrochlorocarbons, hydrogen chloride and both organic and inorganic oxides. Entraining agents have normal boiling points of from about - 110°C to about -25°C. Hydrocarbons include ethane, ethylene, propane, and propylene. Hydrofluorocarbons include methyl fluoride (HFC-41), difluoromethane (HFC-32), 1,1,1-trifluoroethane (HFC-143a), pentafluoroethane (HFC-125), and fluoroethane (HFC-161). Hydrochlorofluorocarbons include chlorodifluoromethane (HCFC-22). Hydrochlorocarbons include methyl chloride (HCC-40). Oxides include nitrous oxide (N₂O), carbon dioxide (CO₂), carbonyl fluoride (COF₂), and perfluoroacetyl fluoride (CF₃COF).

The preferred single component entraining agents for separating PFC-14 and NF₃ by extractive distillation include nitrous oxide (N₂O), chlorodifluoromethane (HCFC-22), difluoromethane (HFC-32), fluoroethane (HFC-161) and methyl fluoride (HFC-41). Most preferred are nitrous oxide and HCFC-22 as extractants for the separation. Although HFC-32, HFC-161 and HFC-41 each require fewer theoretical distillation column stages, lower extractant flow rates, or both, than either nitrous oxide or HCFC-22 to effect an equivalent reduction of PFC-14 in a NF₃ product stream, it was found that nitrous oxide and HCFC-22 are less likely to react in a mixture with NF₃ than HFC-32, HFC-161 and HFC-41.

These single component entraining agents may be used alone or in combination with each other as the extractants for this separation. For example, N₂O forms an azeotropic or azeotrope-like composition with each of HFC-23 and HCl. The respective N₂O/HFC-23 and N₂O/HCl azeotropic or azeotrope-like compositions may each be employed as the extractant for separating NF₃ and PFC-14.

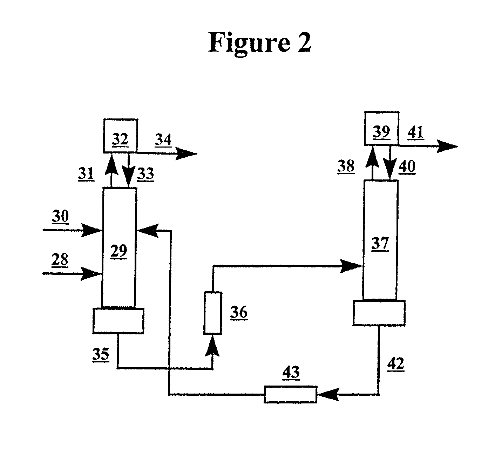

Figure 2 schematically illustrates a system that can be used to perform aspects of the inventive extractive distillation process. A first mixture comprising PFC-14 and NF₃ is supplied via conduit 28 to distillation column 29. At least one extractive agent, e.g., ethane, is supplied via conduit 30 to distillation column 29 at a feed point higher in the column than the feed point of the mixture to be separated, e.g., PFC-14 and NF₃. The overhead distillate from the column is sent via conduit 31 to condenser 32. At least part of the condensed distillate stream is returned to the column 29 as reflux via conduit 33. The remainder of the condensed distillate is recovered via conduit 34 as PFC-14 product substantially free of NF₃ and ethane. A stream comprising ethane and NF₃ substantially free of PFC-14 is removed from the column 29 bottoms via conduit 35 and may be recovered as product. Alternately, the column bottoms stream 35 may be sent to optional cooler 36 and from there fed to distillation column 37, which is operated so as to strip compounds other than the entraining agent from the entraining agent. The distillate from column 37 may be fed via conduit 38 to condenser 39. From condenser 39 some amount of condensed distillate may be returned to the column 37 as reflux via conduit 40, while the remainder recovered as product, e.g., as NF₃ substantially free of PFC-14 and extractive agent, via conduit 41. Extractive agent, e.g., ethane, with the concentration of non-ethane compounds reduced compared to their concentrations in stream 35 is obtained as the distillation column bottoms 42. Stream 42 may optionally be fed to cooler 43 and then returned to distillation column 29 as extractant feed, fed to the column at a feed point higher in the column than that feed point of the mixture to be separated, e.g., PFC-14 and NF₃, or may be optionally mixed with stream 30.

### EXAMPLES

The following Examples are provided to illustrate certain aspects of the present invention, and arc not intended to limit the scope of the invention. The following Examples employ the NRTL equations identified above. In the following Examples, each stage is based upon a 100% operational or performance efficiency. The total stages include condenser and reboiler, with the condenser counted as stage No. 1. In the following Examples, flow rates are given in grams per second, g/s (pounds (weight)-per-hour (pph) or pound-moles-per-hour (mph)); temperatures are expressed in degrees Celsius (°C); pressures are expressed in Pascales, Pa (pound-per-square-inch-absolute (psia)); stream concentrations are expressed in parts-per-million-by-weight (ppmm or ppm-weight) or parts-per-million-by-moles (ppmm or ppm-molar); heat flow rates removed by the condenser or put into the reboiler of the distillation columns are expressed in Watts, W (pcu/hour or pcu/hr).

### COMPARATIVE EXAMPLE 1

In this Comparative Example, a crude feed stream comprising NF₃ and PFC-14 is fed to a distillation column operated under the five sets of conditions (cases) shown in Table 2, with the results of the distillations shown in the respective columns. The distillation columns in these Cases are operated to remove a PFC-14 product from the column as overhead distillate and a NF₃ product as column bottoms.

In Case 1 of this Comparative Example, a crude 12.5 g/s (100 pph) NF₃ feed stream containing 10,000 ppm-weight of PFC-14 is feed to a distillation column. The column has 200 stages. The reflux ratio is approximately 5000:1. As may be seen in this Case, even when 20% of the NF₃ feed to the column is taken overhead, the concentration of PFC-14 in the NF₃ bottoms product is only reduced to 211 ppm-weight.

Compared to Case 1, in Case 2 of this Comparative Example the PFC-14 concentration in the crude NF₃ stream fed to the column has been reduced to 1,000 ppm-weight, the reflux rate has been increased to 62749 g/s (500,000 pph), the distillate takeoff rate has been decreased to 0.06 g/s (0.5 pph), and the bottoms takeoff rate has been increased to 12.5 g/s (99.5 pph). As may be seen in this Case, the PFC-14 concentration in the NF₃ bottoms product is still only reduced to 484 ppm-weight.

Compared to Case 2, in Case 3 of this Comparative Example the overhead distillate rate is increased to 0.63 g/s (5.0 pph) and the bottoms takeoff rate has been decreased to 11.9 g/s (95.0 pph). As may be seen in this Case, the NF₃ bottoms takeoff product still contains 65 ppm-weight PFC-14, even though 5% of the NF₃ feed is being lost in the overhead distillate.

In Case 4 of this Comparative Example, the feed to the distillate column is a 60/40 molar ratio of NF₃/PFC-14, which comprises approximately the azeotropic or azeotrope-like composition formed by NF₃ and PFC-14 at -75 °C. In spite of allowing more than 50% of the NF₃ fed to be removed overhead in the distillate, there is essentially no change in either the distillate or bottoms product compositions compared to the composition of the crude feed stream.

In Case 5 of this Comparative Example, the number of column stages in Case 4 is doubled, and the reflux rate increased. In spite of this, there is still essentially no substantial change in either the distillate or bottoms product composition compared to the composition of the crude feed stream.

The Cases in this Comparative Examples show that, even with many distillation column stages and extremely high reflux ratios, it is impossible to recover a NF₃ product by conventional distillation from a first mixture comprising NF₃ and PFC-14 where said NF₃ is recovered substantially free of PFC-14 and with high recovery efficiency. The NF₃ product purity and recovery efficiency in such distillations is limited because an azeotropic or azeotrope-like composition is formed between NF₃ and PFC-14.

However, Cases 1, 2 and 3 of this Comparative Example do show that by operating a distillation column under conditions such that azeotropic or azeotrope-like compositions of NF₃ and PFC-14 are formed and where said NF₃/PFC-14 azeotropic or azeotrope-like compositions are higher in PFC-14 concentration than the NF₃/PFC-14 concentration in a first mixture, PFC-14 may be removed from the NF₃ of a first mixture. By distilling the NF₃/PFC-14 azeotropic or azeotrope-like composition formed as overhead distillate, an NF₃ product may be recovered as distillation column bottoms where the concentration of the PFC-14 in said NF₃ product has been reduced compared to the first mixture. However, since some portion of the NF₃ in the first mixture necessarily remains with the PFC-14 removed in the distillate, only a limited recovery efficiency of the NF₃ fed in the first mixture is possible.

### EXAMPLE 1

In each Case of this Example, a crude NF₃ feed stream comprising NF₃ and PFC-14 is fed to a distillation column. HCl is also added to the distillation column. The distillation column is operated to recover a PFC-14 product from the column as overhead distillate, while a NF₃ product is recovered as column bottoms. The conditions and the results of the distillations for each Case are shown in Table 3.

In Case 1, the crude NF₃ feed stream comprises NF₃ containing 1000 ppm-weight of PFC-14 and the crude stream feed rate is 100 pph. 1 pph of HCl is fed to the column as an entraining agent. The column has 122 stages and the reflux ratio is 5000:1. The distillate rate is 10 pph, such that approximately 10 % of the NF₃ in the crude feed stream exits overhead with the PFC-14 distillate product. As a result of this distillation, a NF₃ bottoms product is obtained that contains 85 ppm-weight PFC-14.

In Case 2, the number of column stages is increased to 244. As a result of this distillation, a NF₃ bottoms product is obtained that contains 1.2 ppm-weight PFC-14.

In Case 3, the crude NF₃ feed stream instead contains 100 ppm-weight PFC-14 and the reflux rate is 2761 g/s (22,000 pph). As a result of this distillation, a NF₃ bottoms product is obtained that contains about 10 ppm-weight PFC-14.

In Case 4, the distillate takeoff rate is changed to 2.5 g/s (20 pph). As a result of this distillation, a NF₃ bottoms product is obtained that contains about 5 ppm-weight PFC-14.

The Cases of this Example show that by adding or having present HCl in a mixture comprising NF₃ and PFC-14, then by distilling the mixture under conditions sufficient to form the PFC-14/HCl azeotrope, the PFC-14 may be separated from the NF₃. The PFC-14 is recovered in the distillate as the HCl/PFC-14 azeotrope, and a NF₃ product may be recovered as columns bottoms substantially free of PFC-14 and with high recovery efficiency of the NF₃.

### EXAMPLE 2

In each Case of this Example, a crude NF₃ feed stream comprising NF₃ and PFC-14 is fed to a distillation column operated under the conditions shown in Table 4. The concentration of the PFC-14 in the feed stream is 10,000 ppm-molar. A different compound is fed to the column as an extractive agent in each Case. The columns in these Cases are operated to remove PFC-14 from the column as overhead distillate, while recovering a NF₃ product as column bottoms. The results of the distillations are shown in Table 4. In this Example, the concentration of PFC-14 and NF₃ shown in each of the column bottoms streams is calculated versus the total weight or moles of PFC-14 and NF₃ in that stream only, and ignore the weight or moles of any extractant present.

In Table 4, PFC-218 is perfluoropropane or octafluoropropane (C₃F₈); PFC-1 16 is perfluoroethane or hexafluoroethane (C₂F₆), CFC-13 is chlorotrifluoromethane (CClF₃), CFC-115 is chloropentafluoroethane (C₂ClF₅), HFC-125 is pentafluoroethane (C₂HF₅), CO₂ is carbon dioxide, HFC-143a is trifluoroethane (C₂H₃F₃), HFC-23 is trifluoromethane (CHF₃), N₂O is nitrous oxide, C₂H₆ is ethane, HFC-41 is fluoromethane (CH₃F), HCl is hydrogen chloride, HCC-40 is chloromethane (CH₃Cl), HCFC-22 is chlorodifluoromethane (CHClF₂), HFC-32 is difluoromethane (CH₂F₂), HFC-161 is fluoroethane (C₂H₅F).

In Cases 1 and 2, where PFC-218 and PFC-116 are respectively fed as the extractive agent, the concentration of PFC-14 in the NF₃ column bottoms product is essentially unchanged from that of the crude feed stream. For example, Cases 1 and 2 show a PFC-14 concentration of 10,000 ppm-molar in the crude feed stream, while the PFC-14 concentration in the column bottoms is 10,100 ppm-molar with PFC-116 as the extractive agent and 9,110 ppm-molar with PFC-218 as the extractive agent. Cases I and 2 are comparative Cases that show fully-fluorinated compounds such as PFC-218 and PFC-116 are ineffective as extractive agents for separating PFC-14 from NF₃.

By contrast Cases 3 through 16 show their respective extractive agents are effective for separating PFC-14 from NF₃, such that NF₃ having significantly reduced concentrations of PFC-14 versus the crude feed stream may be recovered as a NF₃ product from the distillation column bottoms.

The effectiveness of the extractive agents generally increase from Cases 3 through 16, as indicated by the fact that lower residual PFC-14 concentrations in the NF₃ product bottoms stream are obtained in Cases 3 through 8, and lower extractive agent flow rates on a molar basis are required for Cases 9 through 16 to acheive the same residual PFC-14 concentration in the NF₃ bottoms product. Thus, N₂O, C₂H₆, HFC-41, HCl, HCC-40, HCFC-22, HFC-32 and HFC-161 from Cases 9 through 16, respectively, are more effective than CO₂, HFC-143a and HFC-23 from Cases 6 through 8, respectively, which are in turn more effective than CFC-13, CFC-115 and HFC-125 from Cases 3 through 5, respectively. The extractive agents of Cases 9 through 16 are particularly effective in the separation, producing an NF₃ product containing about 0.1 ppm-molar PFC-14 or less than about 0.13 ppm-weight PFC-14, and in recovering said NF₃ product with greater than 99% recovery efficiency from the crude feed stream.

The extractive agent indicated may optionally be separated from the extractive distillation column bottoms by distillation or other methods disclosed in the present specification, and an NF₃ product that is substantially free of both PFC-14 and extractive agent may be recovered.

### EXAMPLE 3

In the Cases of this Example, a crude feed stream comprising 12.4 g/s (99.19 pph) PFC-14 and 0.10 g/s (0.81 pph) NF₃ is fed to a distillation column. A different entraining agent is fed as an extractant to the columns in each Case. The columns in these Cases are operated to recover a PFC-14 product from the column as overhead distillate, while removing NF₃ in the column bottoms. The extractive agents used and the results of these distillations are shown in Table 5.

The Cases from this Example show that the extractive agents of the current invention are also effective for removing NF₃ from a stream comprising PFC-14 and NF₃ such that a PFC-14 product substantially free of NF₃ may be recovered with high recovery efficiency.

### EXAMPLE 4

In the four Cases of this Example, a crude feed stream comprising 5.65 g/s (45 pph) NF₃ and 6.90 g/s (55 pph) PFC-14 is fed to distillation columns having the same number of stages, operated at the same pressure, with extractive agent flow rate and feed points in the column chosen to give the desired separation. In all Cases the distillation is operated so that a NF₃ product is recovered as distillation column bottoms with a PFC-14 product removed as overhead distillate. N₂O is fed as the extractive agent in Cases 1 and 3, and HCFC-22 is fed as the extractive agent in Cases 2 and 4. The results of these distillations are shown in Table 6.

As may be seen, Cases 1 and 2 recover a NF₃ bottoms product stream in which the PFC-14 concentration is 0.1 ppm-molar versus the NF₃ in that stream, with the NF₃ recovery being about 96% of the NF₃ originally fed. Cases 3 and 4 recover a PFC-14 overhead distillate product stream in which the NF₃ concentration is 0.1 ppm-molar versus the PFC-14 in that stream, with that PFC-14 recovery being about 98% of the PFC-14 originally fed.

This Example shows the versatility of the instant extractive distillation invention, where the same distillation column can be employed to produce either PFC-14 substantially free of NF₃ or produce NF₃ substantially free of PFC-14, each with high recovery efficiency, from the same feed stream, and even when said feed stream contains high concentrations of the component that needs to be removed. Changing the operation of the column to alternate between recovering higher purity NF₃ or higher purity PFC-14 as product is effected simply by changing the crude feed and extractive agent feed points on the column, raising the feed and extractive agent feed points for high purity NF₃ product recovery, lowering the feed and extractive agent feed points for high-purity PFC-14 product recovery, and by adjusting the reflux rate, extractive agent feed rate, and crude fed rate as necessary to acheive 99.9999% or greater product purity.

### EXAMPLE 5

In the Cases of this Example, a crude feed stream comprising 12.4 g/s (98.76 pph) NF₃ and 0.16 g/s (1.24 pph) PFC-14 is fed to one or more distillation columns. The conditions and results of the distillations are shown in Table 7 (conventional distillation) and in Table 8 (extractive distillation).

Case 5 shown in Table 8 illustrates the complete extractive distillation process, including extraction column, stripping column and extractive agent feed cooler as shown in Figure 2. N₂O is used as the extractive agent. Case 5 is similar to Case 9, Table 4, which also used N₂O as the extractive agent. In this Example, Case 5, the stripping column tails stream, which contains small amounts of PFC-14 and NF₃ is recycled back to the top of the extraction column. As can be seen in Table 8, the extraction column bottoms stream contains 0.1 ppm-molar PFC-14 versus NF₃, just as it did in Case 9, Table 4. The extraction column bottoms stream is fed to the stripping column where the NF₃ product is removed overhead in the distillate stream, still containing 0.1 ppm-molar PFC-14 and non-detectable amounts of the extractive agent N₂O. This Example illustrates the operation of the stripping column and it demonstrates it is possible to produce NF₃ product which is substantially free of all other impurities (NF₃ purity 99.9999% or higher).

Case 1 in Table 7 uses a single distillation column with 195 stages, which is the same as the total number of stages for the combined extraction and stripping columns in Case 5, Table 8. No entraining agents are added. Case 1 in Table 7 has a total condenser refrigeration duty of -51434 W (-97500 pcu/hour), which is the same as the total combined refrigeration cooling duty for the extraction and stripping column condensers plus the recycle extractant cooler in Case 5, Table 8. In Case 1, the column is operated to remove PFC-14 in the overhead distillate and the NF₃ product as the column bottoms. The distillation produces a NF₃ product containing slightly reduced PFC-14 concentration, 7298 ppm-molar, versus the PFC-14 concentration in the crude column feed stream, 10000 ppm-molar. In Case 2, the conditions are similar to Case 1, but the distillate takeoff rate is increased ten times. The NF₃ bottoms product in Case 2, however, only decreases slightly in PFC-14 concentration compared to Case 1, from 7298 ppm-molar to 1969 ppm-molar. In Case 3, the conditions are similar to Case 1, but the reflux condenser duty is increased ten times. The PFC concentration in the NF₃ bottoms product in Case 3 again decreases only slightly compared to that of Case 1, from 7298 ppm-molar to 6253 ppm-molar. In Case 4, both the reflux condenser duty and the distillate rate are each increased ten times compared to Case 1. However, the NF₃ bottoms product still contains 287 ppm-molar PFC-14.

Cases 1 through 4 of this Example are comparative examples showing conventional distillation is ineffective in producing a NF₃ product stream that is substantially free of PFC-14. Case 5, which uses extractive distillation, clearly shows the significant improvement provided by the current invention in producing high-purity NF₃ product versus conventional techniques.

### EXAMPLE 6

In this Example, a crude feed stream comprising 12.4 g/s (98.76 pph) NF₃ and 0.16 g/s (1.24 pph) PFC-14 is fed to two distillation columns in an extractive distillation process which uses HCl as the extractive agent. The conditions and results of the distillations are shown in Case 1, Table 9. This Case is similar to Case 12, Table 4, which also used HCl as the extractive agent. This Case illustrates the complete extractive distillation process, including extraction column, stripping column and extractant feed cooler as shown in Figure 2, and it also illustrates the use of the HCl/NF₃ azeotrope in a NF₃ purification process.

In the Example, the extraction column is operated under the same conditions as in Case 12, Table 4. The extraction column bottoms stream is fed to a stripping column operated under conditions to form an HCl/NF₃ azeotropic or azeotrope-like composition. Forming the the azeotropic or azeotrope-like composition allows NF₃ to be removed from the stripping column as overhead distillate such that an HCl product is recovered from the stripping column bottoms where said HCl product has a NF₃ concentration which is substantially reduced compared to the column feed stream. With this reduction in NF₃ concentration, this HCl product stream may then be recycled as extractive agent to the extraction column. Further, this reduces the volume of HCl that would otherwise be lost if it were removed by other conventional methods, such as if the HCl were removed from the NF₃ by water washing as might have been done with the extraction column bottoms stream of Case 12, Table 4.

By comparing this Example with Case 5 in Table 8, in which N₂O is used as the extractive agent instead of HCl, it can be seen the use of HCl results in equivalent NF₃ product purity (>99.9999%) with fewer total column stages (152 versus 195) and with lower total refrigeration duty for the extraction and stripping columns and recycle cooler (-21734 W (-41200 pcu/hr)) versus (-51434 W (-97500 pcu/hr)). This further illustrates the effectiveness of the HCl/NF₃ azeotrope in the purification of NF₃.

### EXAMPLE 7

This Example demonstrates the existence of azeotropic or azeotrope-like compositions between the binary pairs mixtures consisting essentially of PFC-14 and NF₃; PFC-14 and HCl; NF₃ and HCl; N₂O and HFC-23; and HCl and N₂O.

To determine the relative volatility of each binary pair, the PTx Method was used. In this procedure, for each binary pair, the total absolute pressure in a PTx cell of known volume was measured at constant temperature for various known binary compositions. These measurements were then reduced to equilibrium vapor and liquid compositions using the NRTL equation.

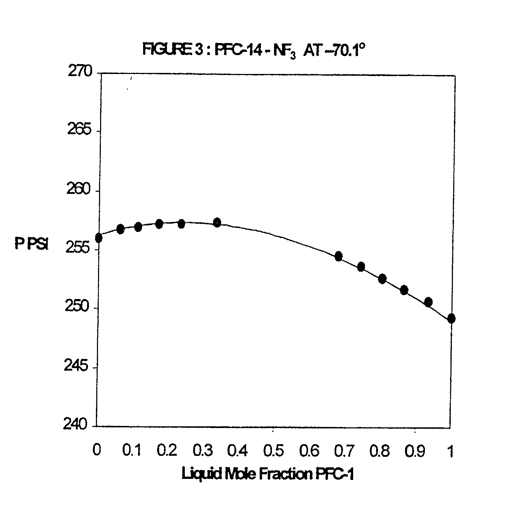

The vapor pressure measured versus the composition in the PTx cell for these binary systems are shown in Figures 3 through 7, respectively. The experimental data points are shown in each Figure as solid points and the solid line is drawn from data calculated using the NRTL equation.

Referring now to Figure 3, Figure 3 illustrates graphically the formation of an azeotropic and azeotrope-like composition consisting essentially of PFC-14 and NF₃ at -70.1°C, as indicated by a mixture of about 36 mole % PFC-14 and 64 mole % NF₃ having the highest pressure over the range of compositions at this temperature. Based upon these findings, it has been calculated that an azeotropic or azeotrope-like compositions of about 20 mole % PFC-14 and 80 mole % NF₃ is formed at -110°C and 0.32 MPa (47 psia), and an azeotropic or azeotrope-like composition of about 55 mole % PFC-14 and 45 mole % NF₃ is formed at -40°C and 4.45 MPa (645 psia). Accordingly, the present invention provides an azeotropic or azeotrope-like composition consisting essentially of from about 20 to about 55 mole % PFC-14 and from about 80 to about 45 mole % NF₃, said composition having a boiling point of from about -110°C at 0.32 MPa (47 psia) to about -40°C at 4.45 MPa (645 psia).

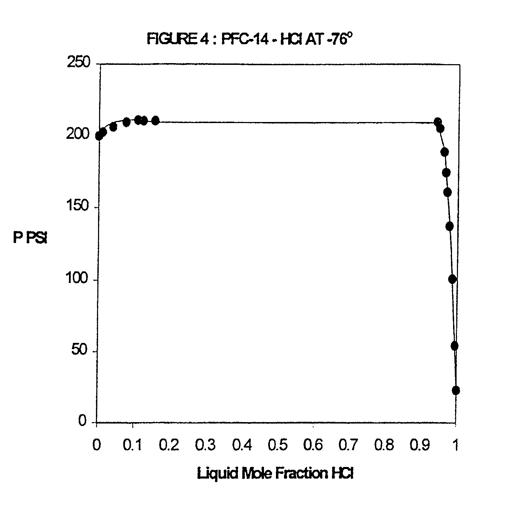

Referring now to Figure 4, Figure 4 illustrates graphically the formation of an azeotropic and azeotrope-like composition consisting essentially of PFC-14 and HCl at -76°C as indicated by a mixture of 91 mole% PFC-14 and 9 mole% HCl having the highest pressure over the range of compositions at this temperature. Based upon these findings, it has been calculated that an azeotropic or azeotrope-like compositions of about 93 mole % PFC-14 and 7 mole % HCl is formed at -100°C and 0.53 MPa (77 psia) and an azeotropic or azeotrope-like composition of about 91 mole % PFC-14 and 9 mole % HCl is formed at -50°C and 3.43 MPa (497 psia). Accordingly, the present invention provides an azeotropic or azeotrope-like composition consisting essentially of from about 93 to about 91 mole % PFC-14 and from about 7 to about 9 mole % HCl, said composition having a boiling point of from about -100°C at 0.53 MPa (77 psia) to about -50°C at 3.43 MPa (497 psia).

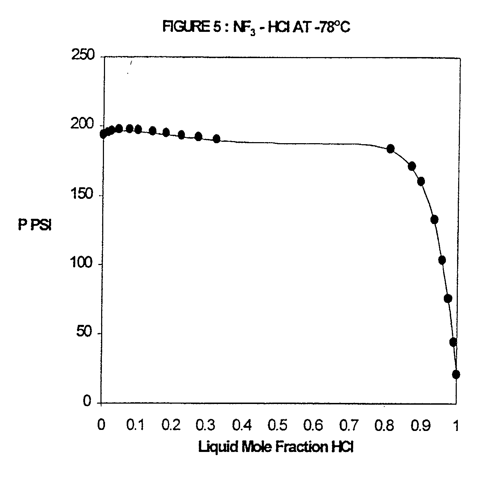

Referring now to Figure 5, Figure 5 illustrates graphically the formation of an azeotropic and azeotrope-like composition consisting essentially of NF₃ and HCl at -78°C, as indicated by a mixture of about 93 mole % NF₃ and 7 mole % HCl having the highest pressure over the range of compositions at this temperature. Based upon these findings, it has been calculated that an azeotropic or azeotrope-like compositions of about 94 mole % NF₃ and 6 mole % HCl is formed at -100°C and 0.54 MPa (79 psia) and an azeotropic or azeotrope-like composition of about 93 mole % NF₃ and 7 mole % HCl is formed at -50°C and 3.36 MPa (487 psia). Accordingly, the present invention provides an azeotropic or azeotrope-like composition consisting essentially of from about 94 to about 93 mole % NF₃ and from about 6 to about 7 mole % HCl, said composition having a boiling point of from about -100°C at 0.54 MPa (79 psia) to about -50°C at 3.36 MPa (487 psia).

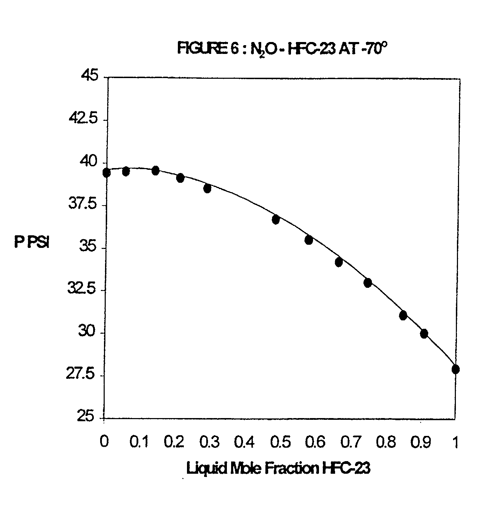

Referring now to Figure 6, Figure 6 illustrates graphically the formation of an azeotropic and azeotrope-like composition consisting essentially of N₂O and HFC-23 at -70°C, as indicated by a mixture of about 94 mole % N₂O and 6 mole % HFC-23 having the highest pressure over the range of compositions at this temperature. Based upon these findings, it has been calculated that an azeotropic or azeotrope-like compositions of about 95 mole % N₂O and 5 mole % HFC-23 is formed at -90°C and 0.09 MPa (13 psia) and an azeotropic or azeotrope-like composition of about 90 mole % N₂O and 10 mole % HFC-23 is formed at 25 °C and 5.68 MPa (824 psia). Accordingly, the present invention provides an azeotropic or azeotrope-like composition consisting essentially of from about 95 to about 90 mole % N₂O and from about 5 to about 10 mole % HFC-23, said composition having a boiling point of from about -90°C at 0.09 MPa (13 psia) to about 25°C at 5.68 MPa (824 psia).

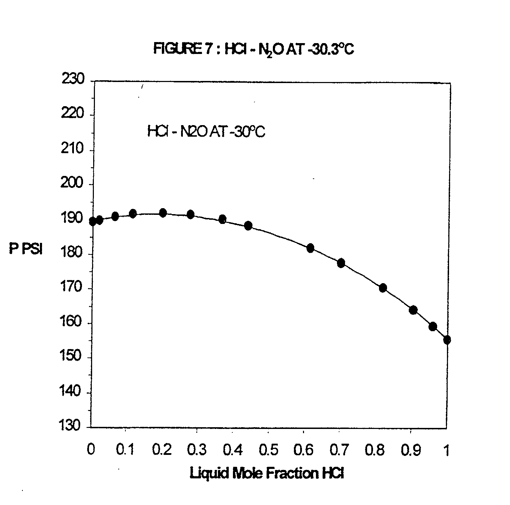

Referring now to Figure 7, Figure 7 illustrates graphically the formation of an azeotropic and azeotrope-like composition consisting essentially of N₂O and HCl at -30.3°C, as indicated by a mixture of about 82 mole % N₂O and 18 mole % HCl having the highest pressure over the range of compositions at this temperature. Based upon these findings, it has been calculated that an azeotropic or azeotrope-like compositions of about 76 mole % N₂O and 24 mole % HCl is formed at -90°C and 0.10 MPa (14 psia) and an azeotropic or azeotrope-like composition of about 83 mole % N₂O and 17 mole % HCl is formed at 25°C and 5.71 MPa (828 psia). Accordingly, the present invention provides an azeotropic or azeotrope-like composition consisting essentially of from about 76 to about 83 mole % N₂O and from about 24 to about 17 mole % HCl, said composition having a boiling point of from about -90°C at 0.10 MPa (14 psia) to about 25°C at 5.71 MPa (828 psia).

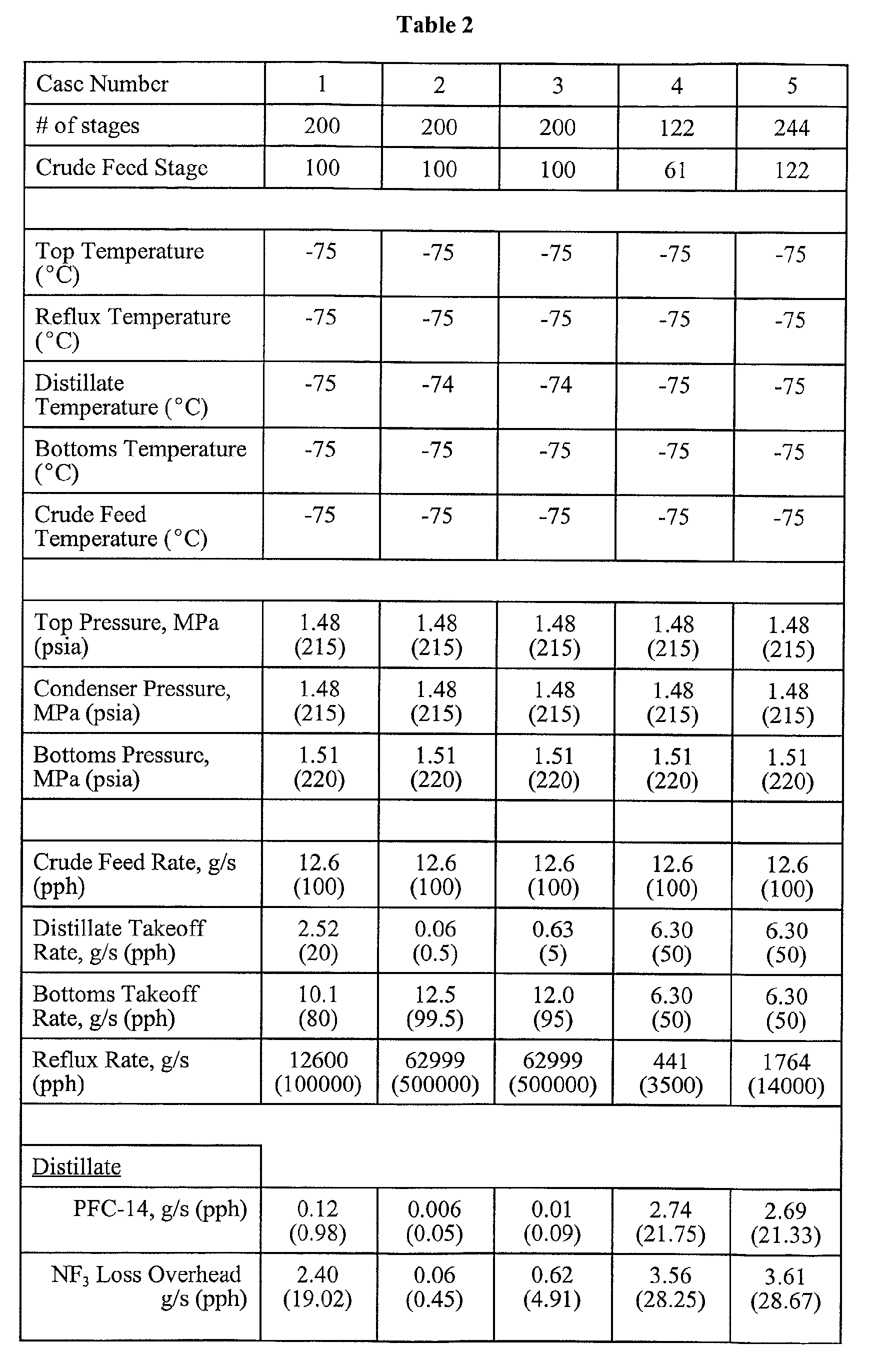

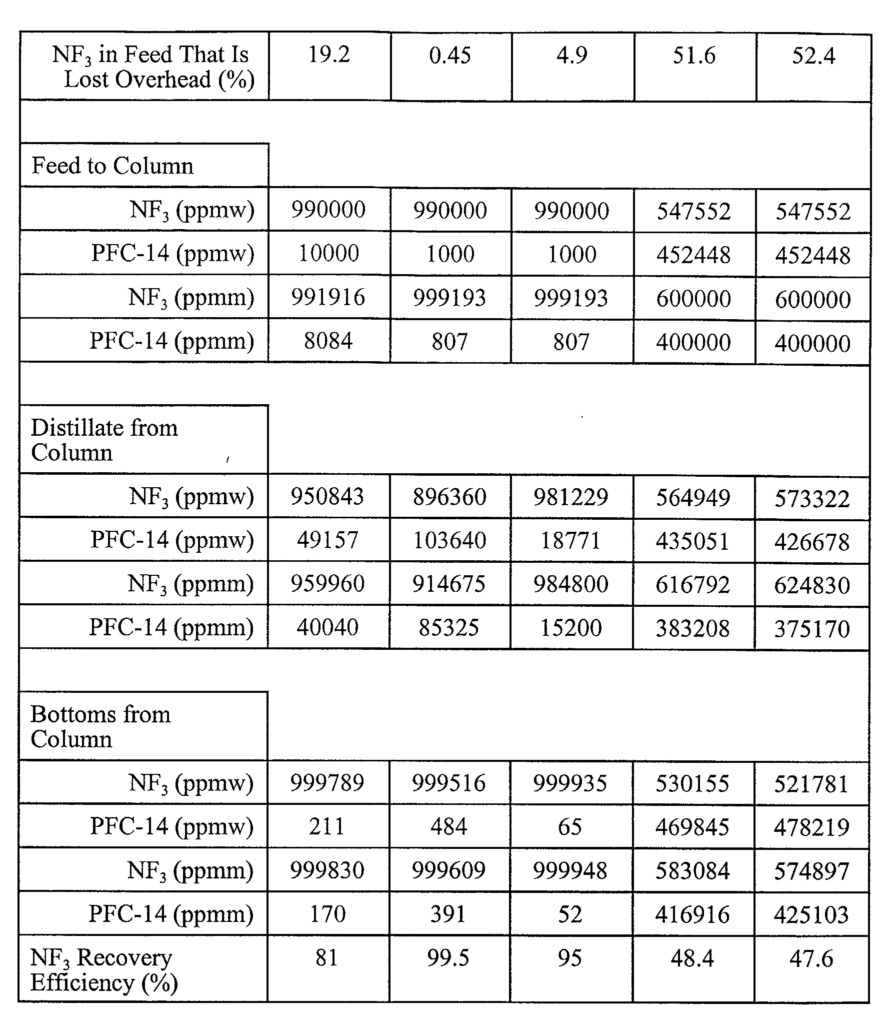

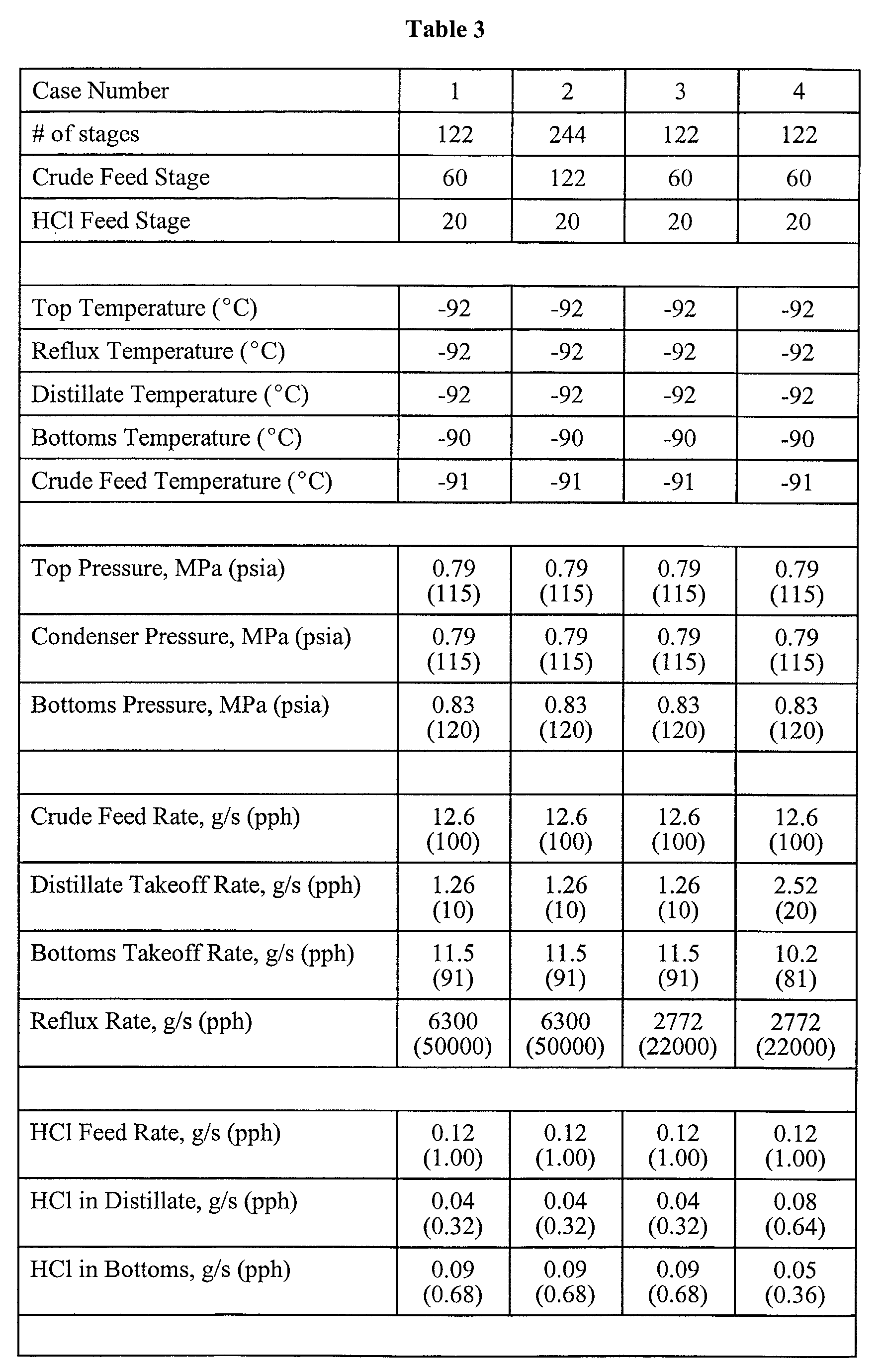

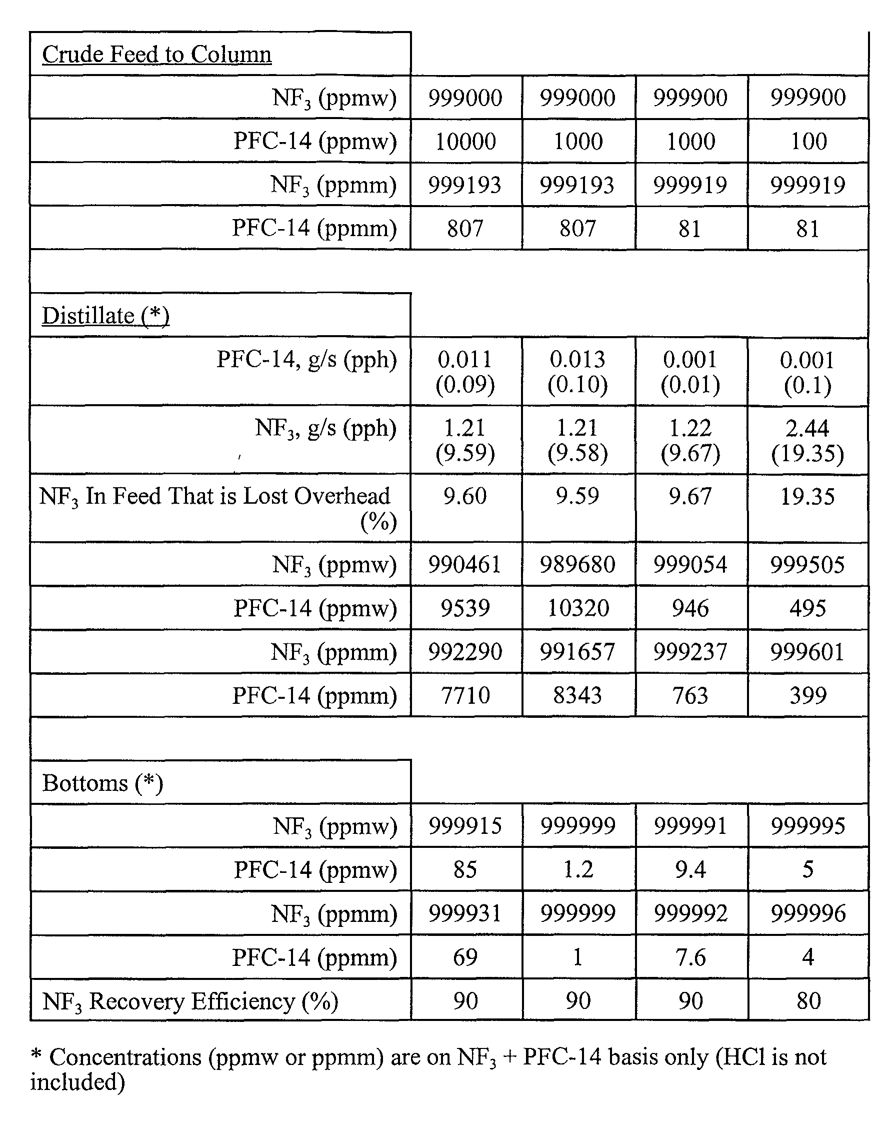

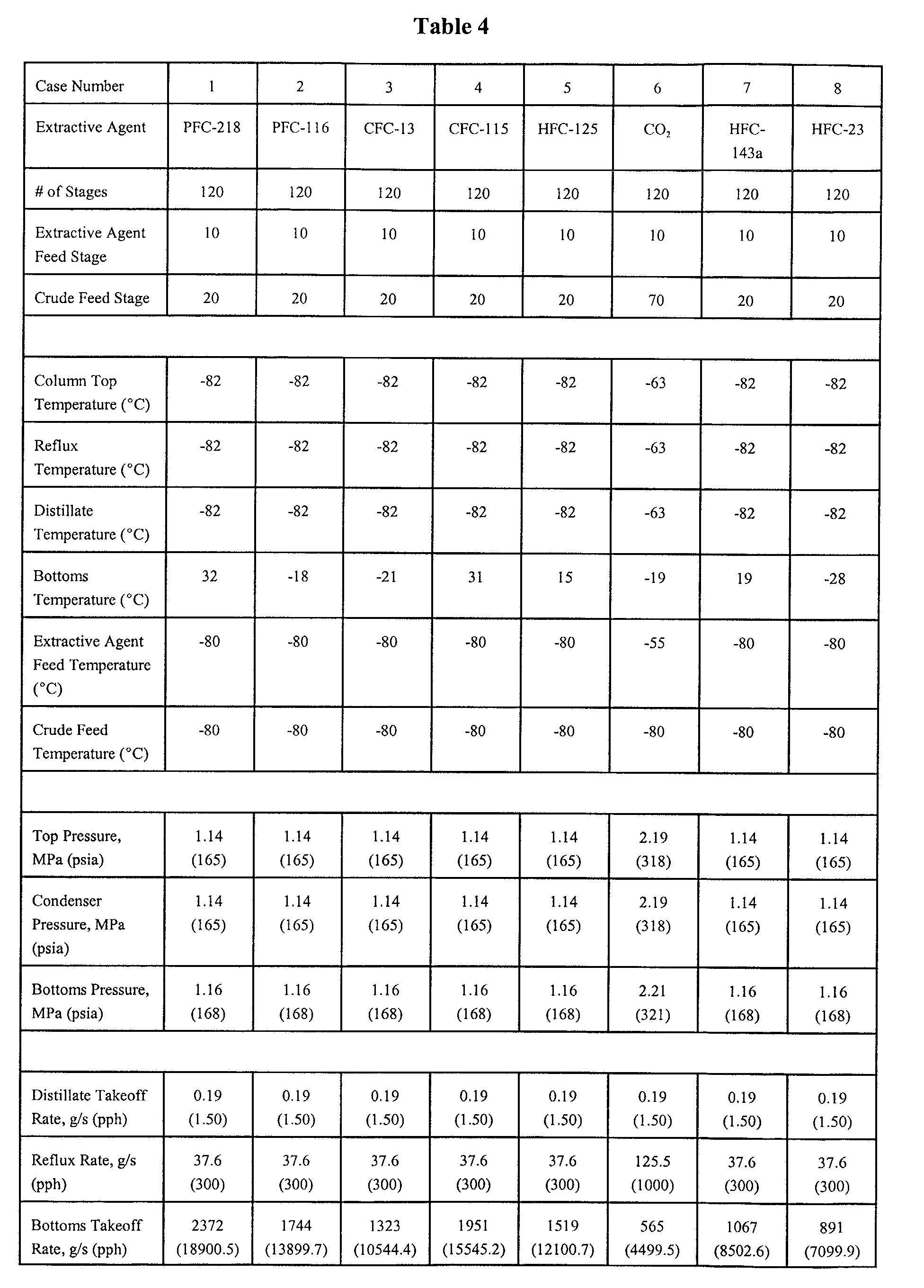

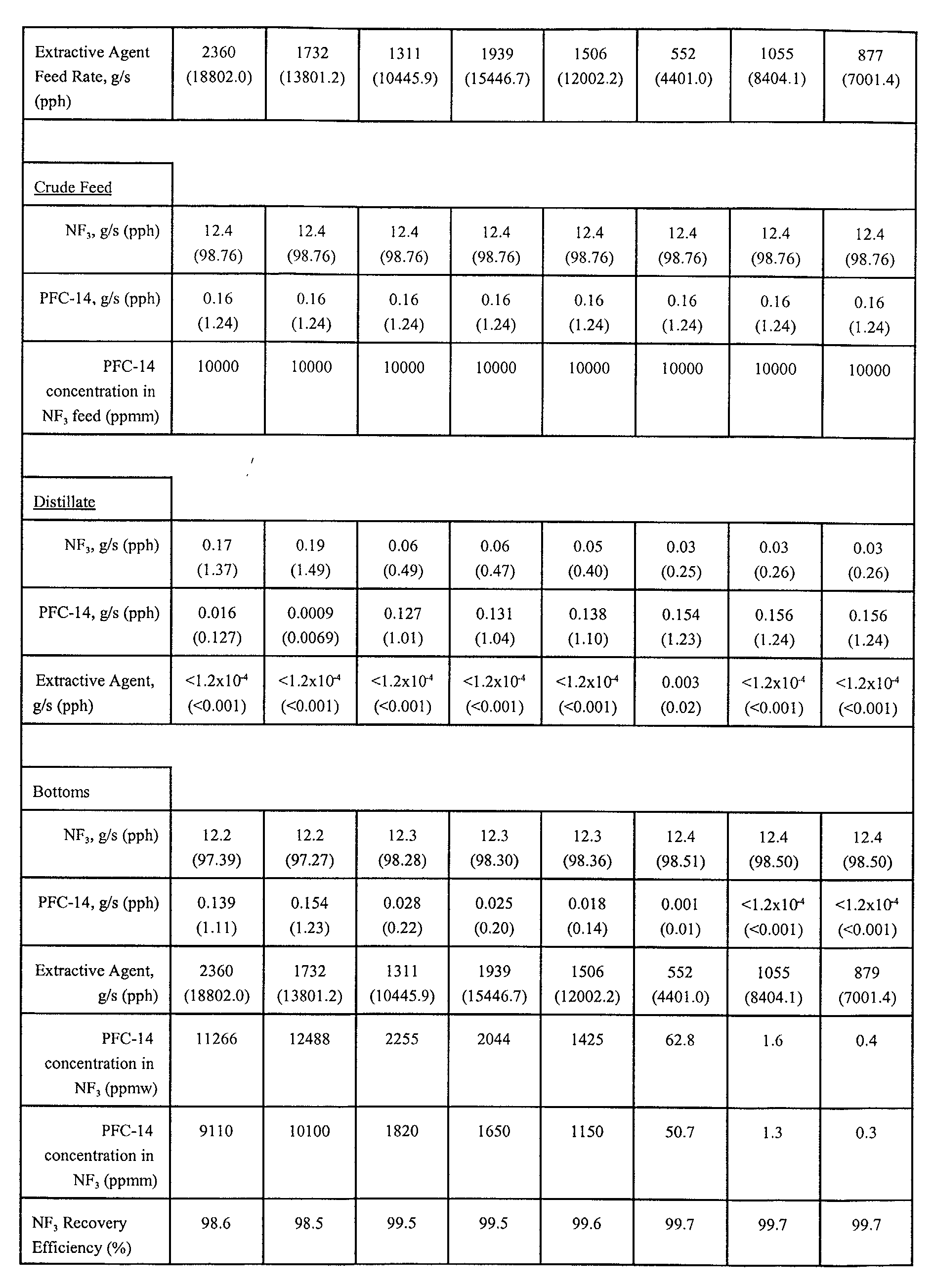

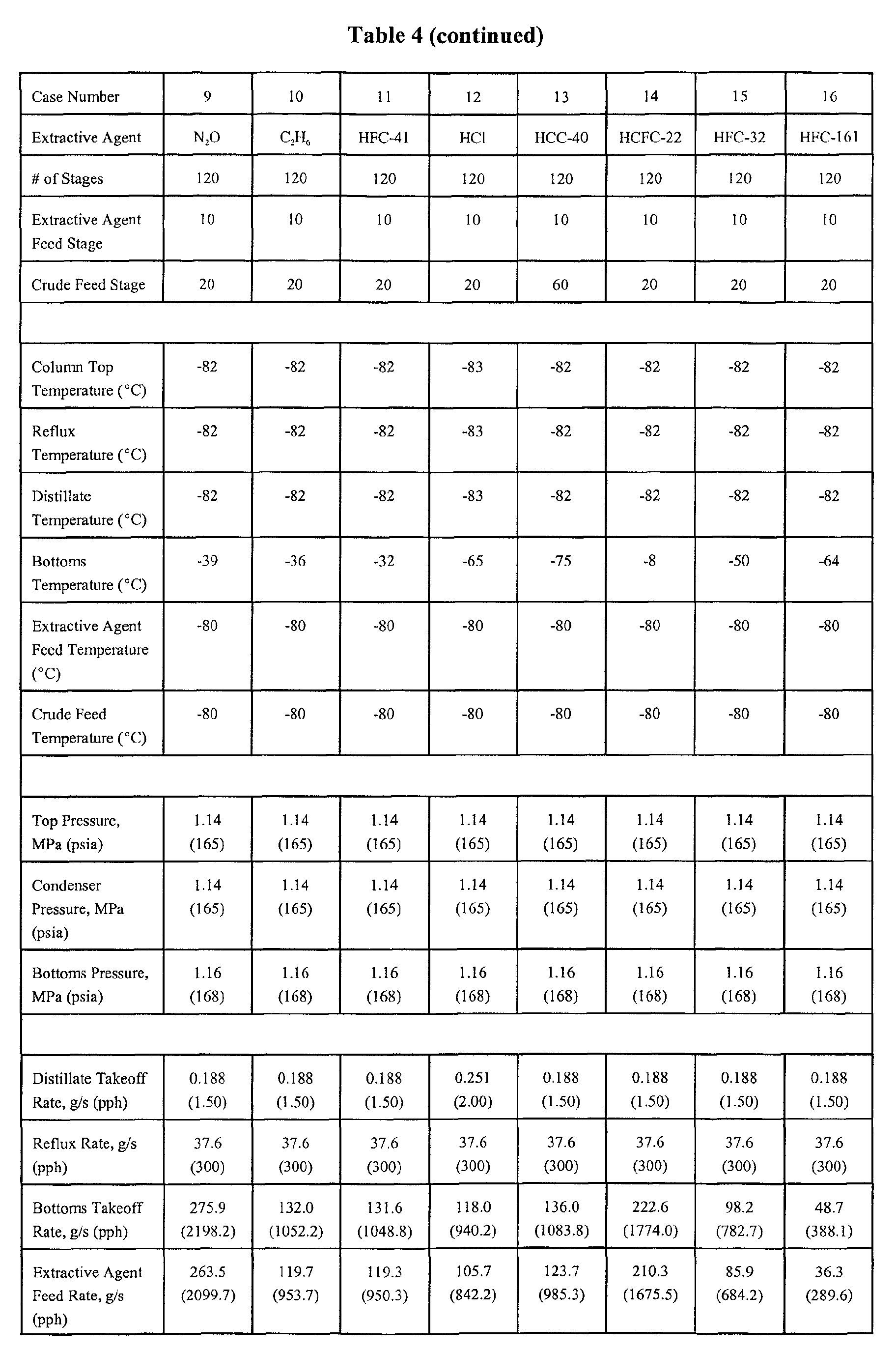

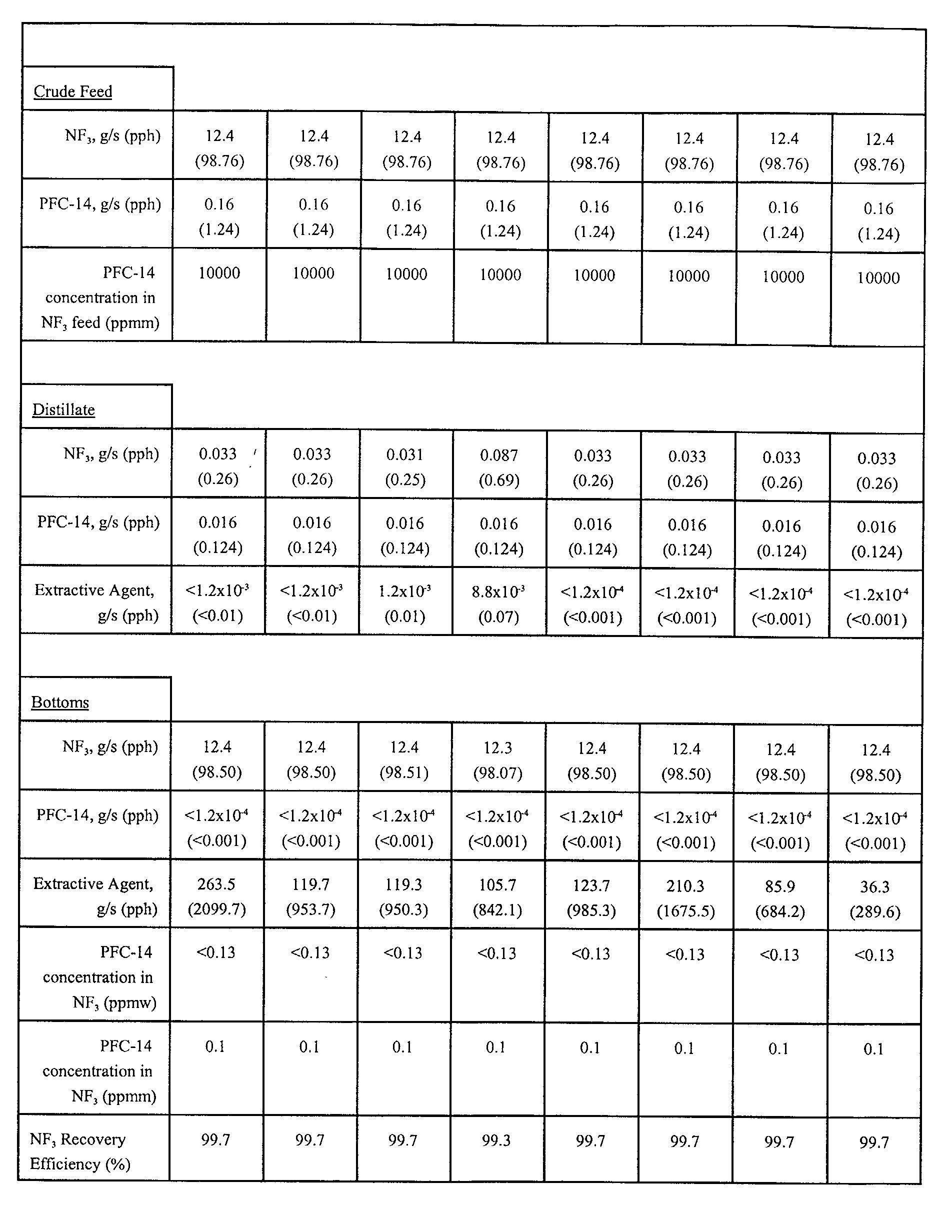

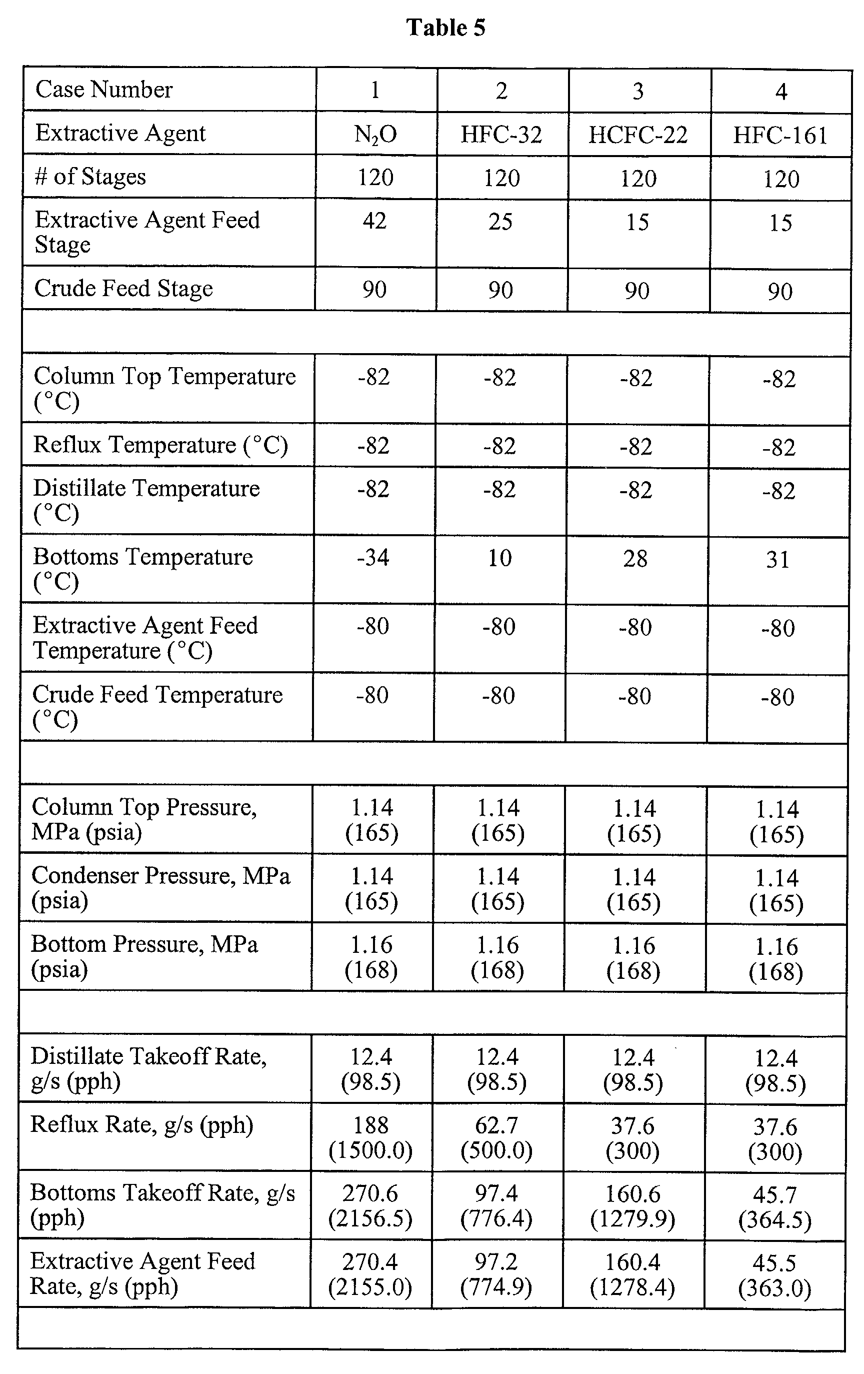

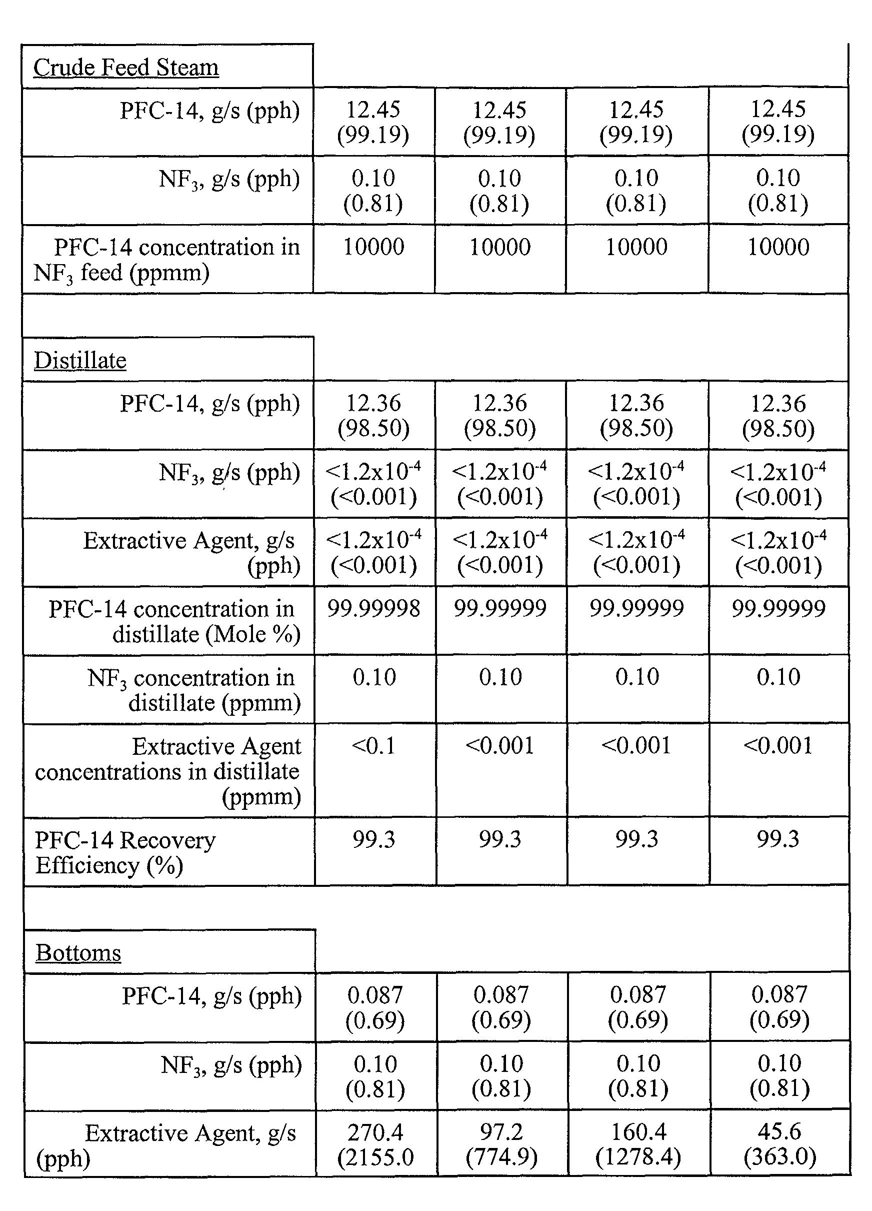

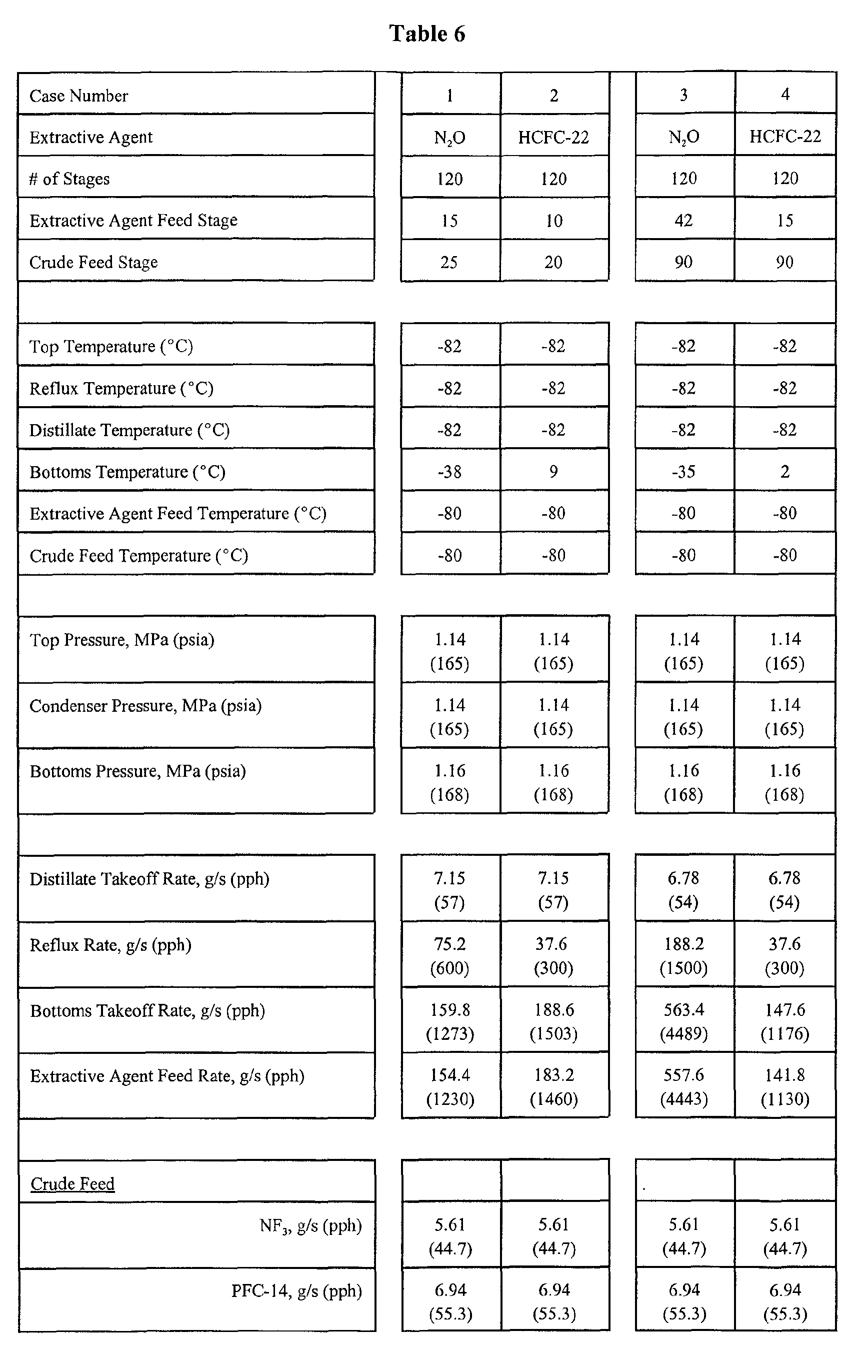

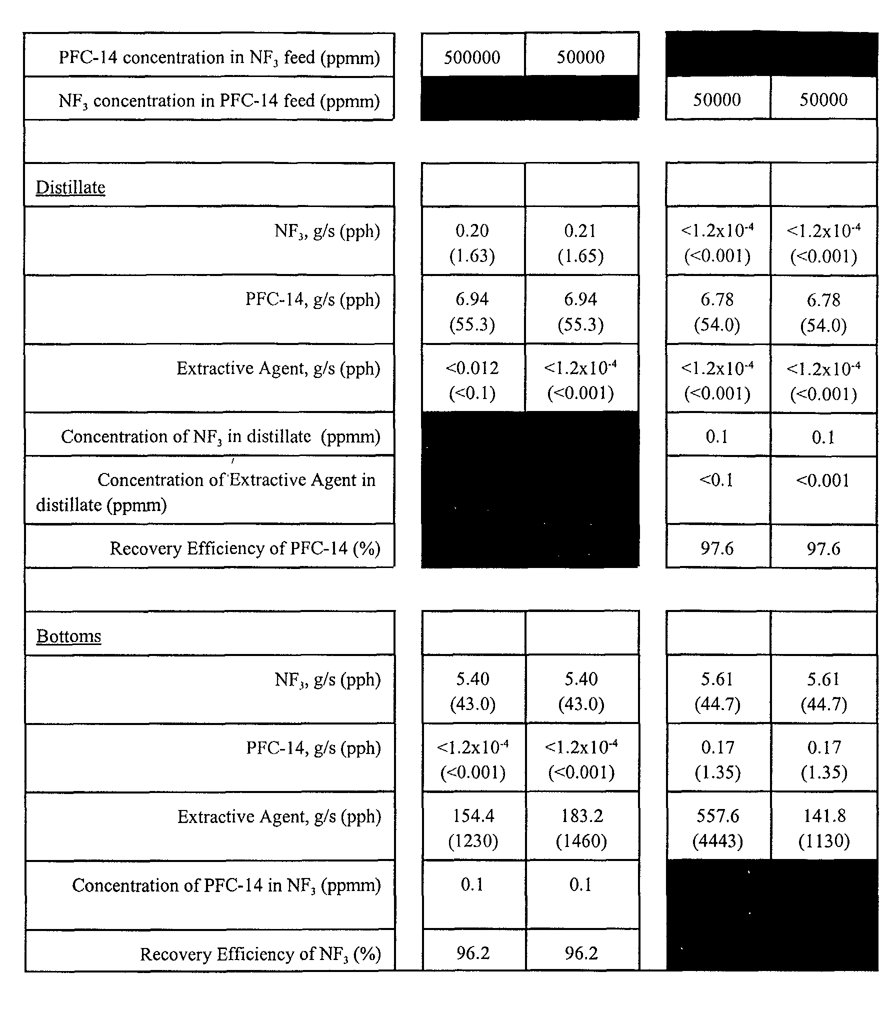

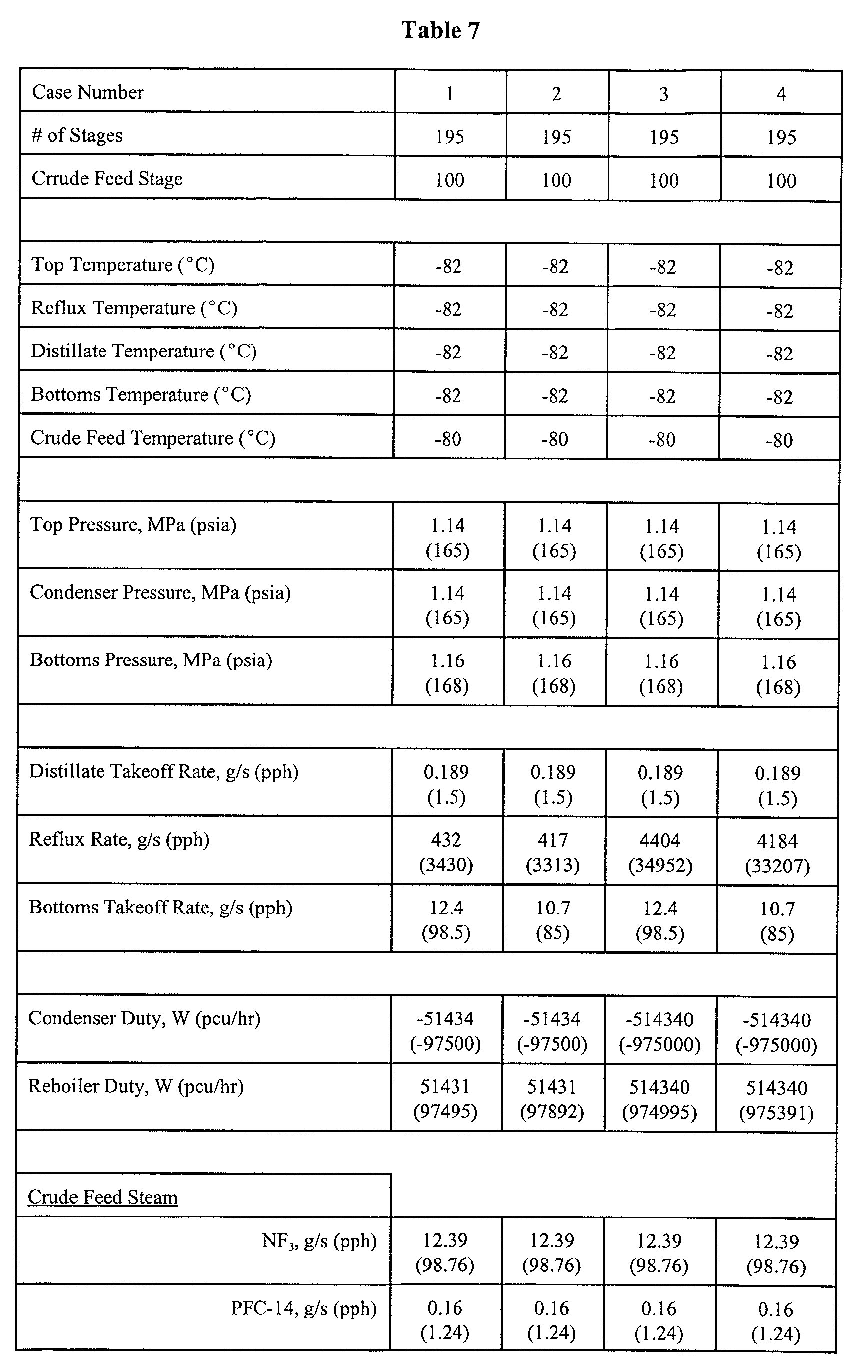

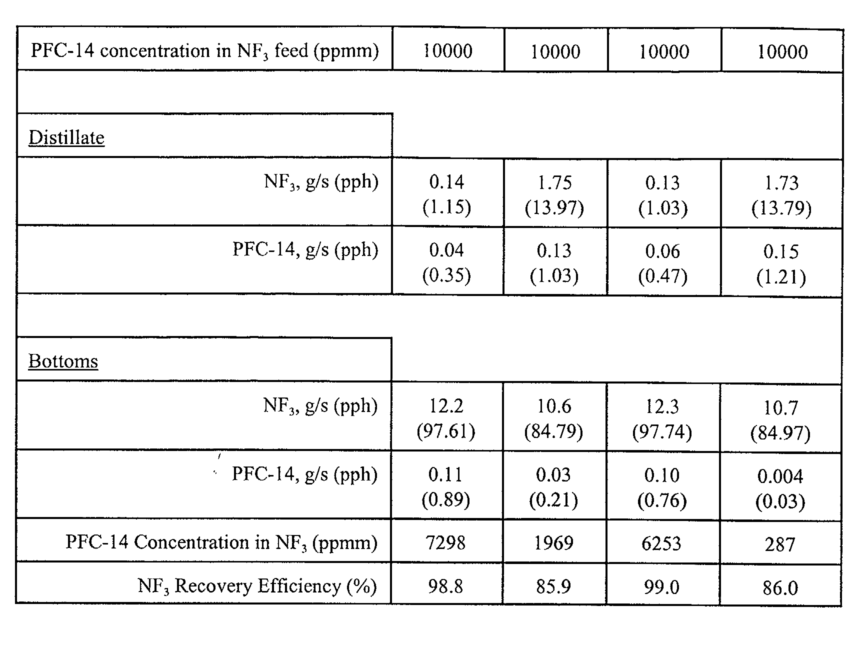

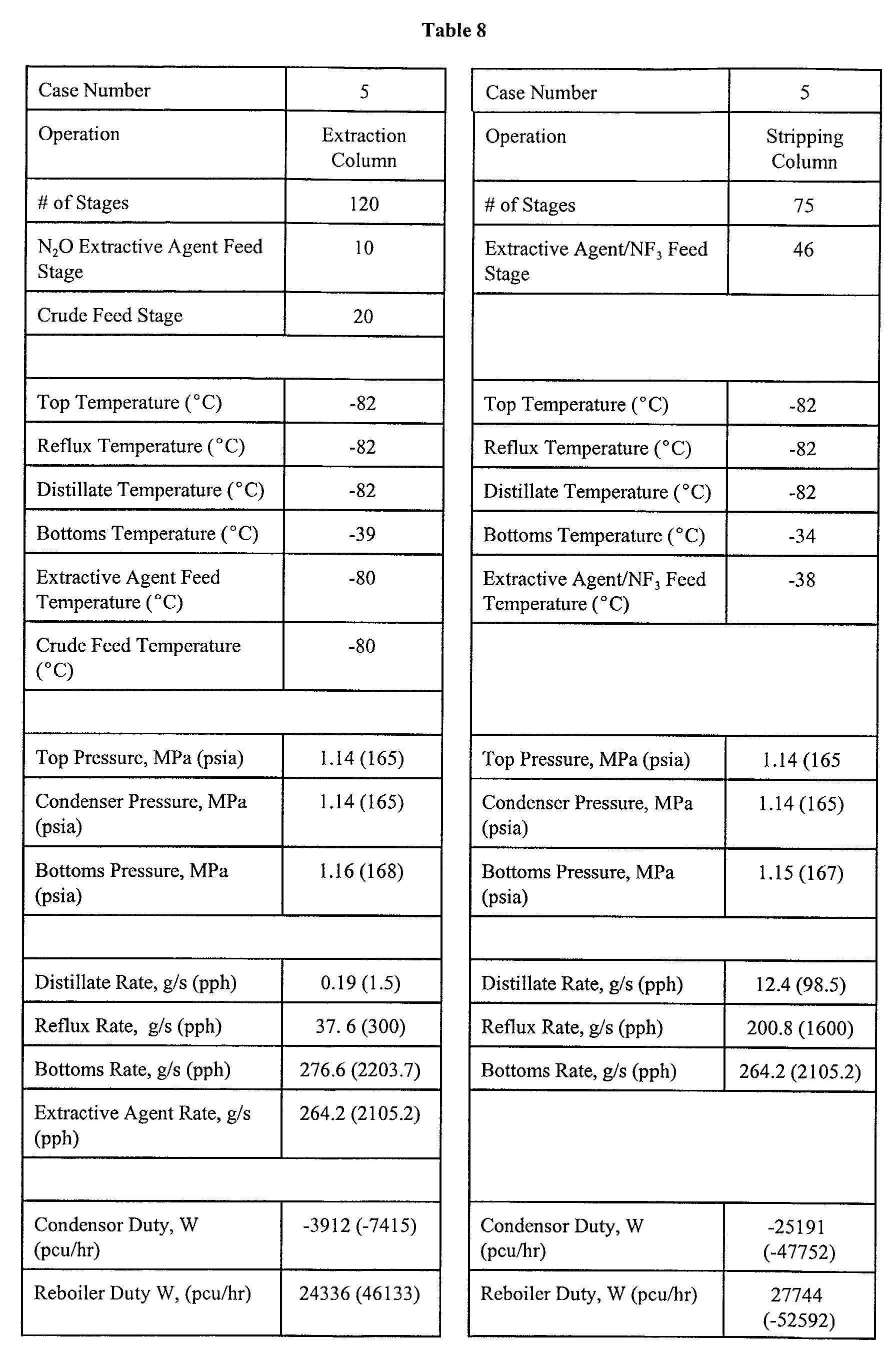

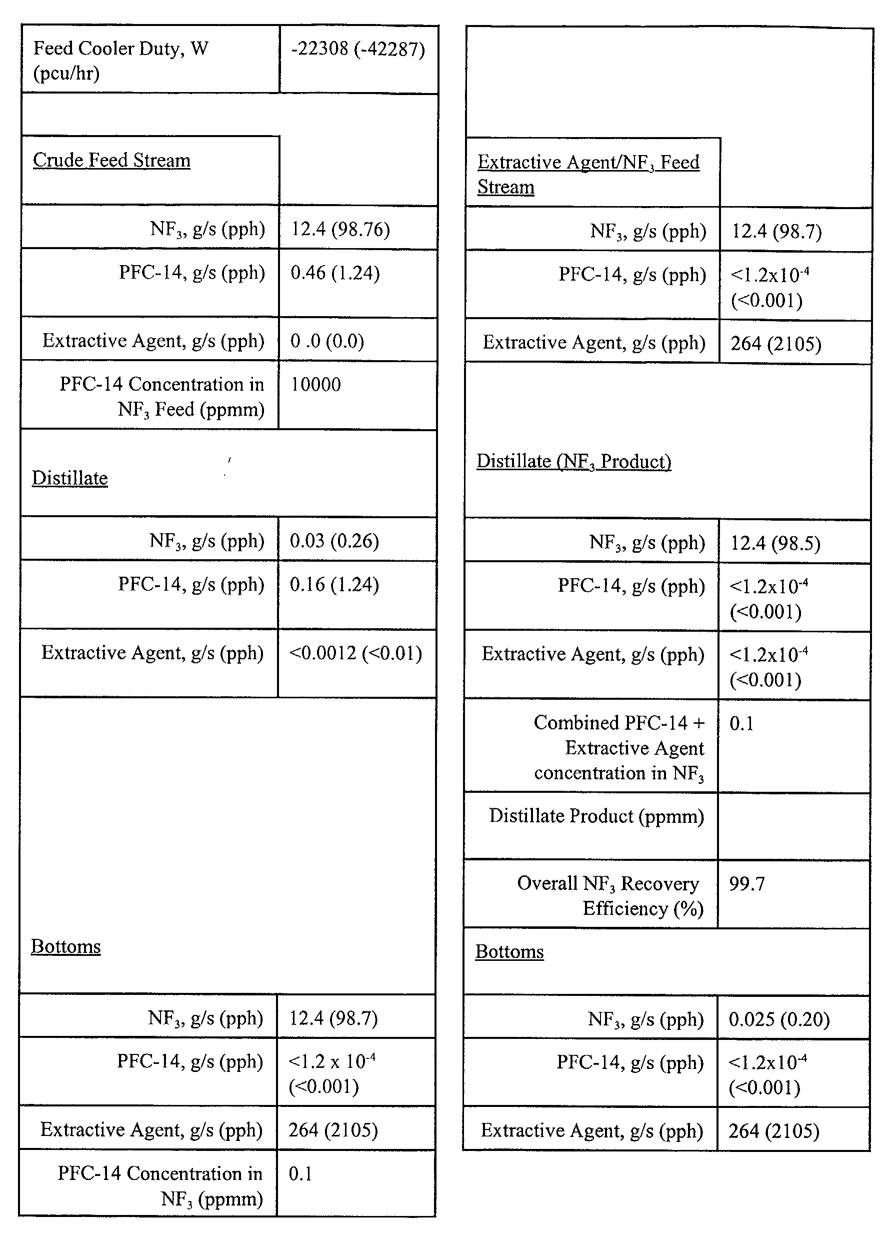

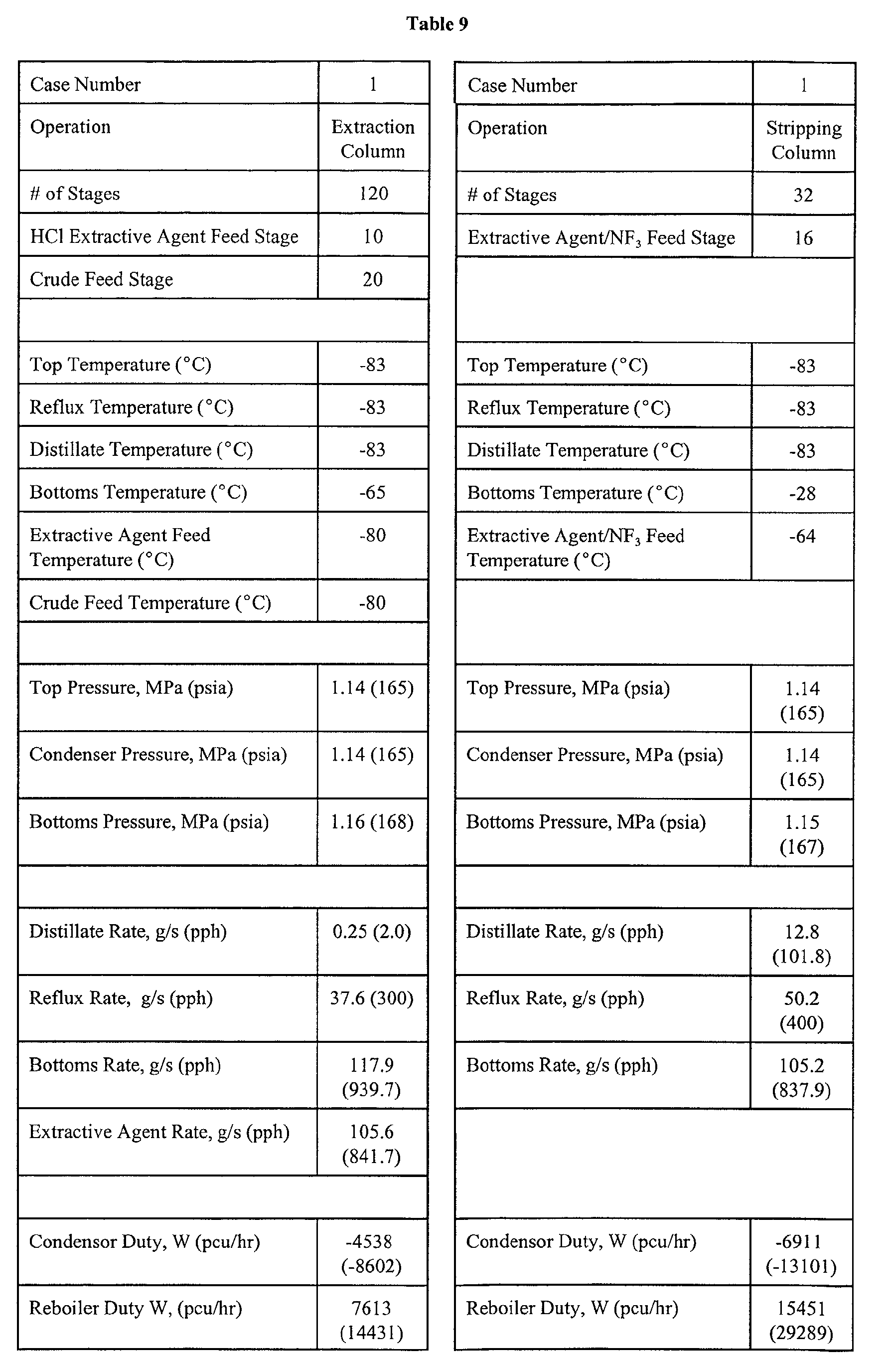

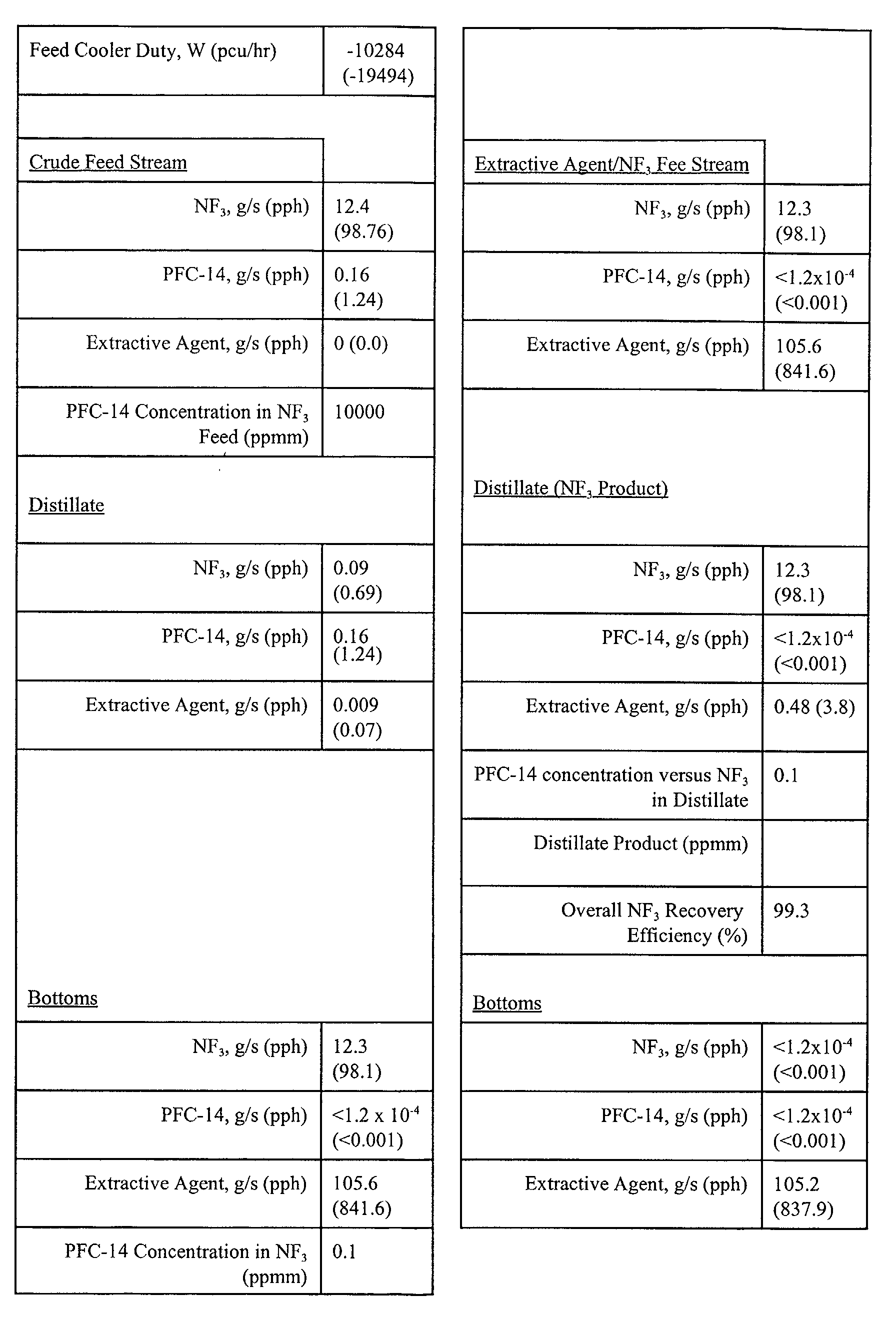

## Claims

1. A process for separating nitrogen trifluoride (NF₃) from a first mixture of nitrogen trifluoride (NF₃) and tetrafluoromethane (PFC-14) wherein the amount of nitrogen trifluoride (NF₃) in the first mixture is in excess of the amount of nitrogen trifluoride (NF₃) in an azeotropic or azeotrope-like composition of nitrogen trifluoride (NF₃) and tetrafluoromethane (PFC-14), comprising:
a.) distilling the first mixture to form an azeotropic or azeotrope-like composition of nitrogen trifluoride (NF₃) and tetrafluoromethane (PFC-14) as a second mixture, and
b.) recovering the second mixture as a distillation column overhead stream, and nitrogen trifluoride (NF₃) as a distillation column bottom stream.

2. A process for separating tetrafluoromethane (PFC-14) from a first mixture of nitrogen trifluoride (NF₃) and tetafluoromethane (PFC-14) wherein the amount of tetrafluoromethane (PFC-14) in the first mixture is in excess of the amount of tetrafluoromethane (PFC-14) in an azeotropic or azeotrope-like composition of nitrogen trifluoride (NF₃) and tetrafluoromethane (PFC-14), comprising:
distilling the first mixture to form an azeotropic or azeotrope-like composition comprising nitrogen trifluoride (NF₃) and tetrafluoromethane (PFC-14) as a second mixture, and
recovering the second mixture as a distillation column overhead stream, and tetrafluoromethane (PFC-14) as a distillation column bottom stream.

3. The process of claims 1 or 2 wherein said said azeotropic or azeotrope-like composition consists essentially of from 20 to 55 mole percent tetrafluoromethane (PFC-14) and from 80 to 45 mole percent nitrogen trifluoride (NF₃), said composition having a boiling point from -110°C at 0.32 MPa (47 psia) to -40°C at 4.45 MPa (645 psia).
